# EUROPEAN PATENT APPLICATION

(11) **EP 4 202 010 A1**
(43) Date of publication of application: **28.06.2023**
(21) Application number: 21864669.3
(22) Date of filing: 02.09.2021
(51) Int. Cl.: C09K 11/06, H01L 51/00, H01L 51/50, H01L 27/32

(54) **POLYCYCLIC AROMATIC DERIVATIVE COMPOUND AND ORGANOELECTROLUMINESCENT DEVICE USING SAME**

(30) Priority: 04.09.2020 KR 20200112964; 01.09.2021 KR 20210116188
(71) Applicant: SFC Co., Ltd., Cheongju-si, Chungcheongbuk-do 28122 (KR)
(72) Inventor: SHIN, Bong-ki, Cheongju-si Chungcheongbuk-do 28122 (KR); JOO, Sung-hoon, Cheongju-si Chungcheongbuk-do 28122 (KR); YANG, Byung-sun, Cheongju-si Chungcheongbuk-do 28122 (KR); KIM, Ji-hwan, Cheongju-si Chungcheongbuk-do 28122 (KR); JO, Hyeon-jun, Cheongju-si Chungcheongbuk-do 28122 (KR); CHOI, Sung-eun, Cheongju-si Chungcheongbuk-do 28122 (KR)
(74) Representative: Grosse, Felix Christopher
(86) International application number: PCT/KR2021/011828
(87) International publication number: WO 2022/050710

(57) **Abstract**

The present invention relates to a polycyclic aromatic derivative compound and a high efficiency and long lifetime organoelectroluminescent device which has significantly improved luminous efficiency by using same. The present invention can implement an organoelectroluminescent device with high efficiency and long lifetime, and thus can be effectively used for industrial applications including flat display devices, flexible display devices, monochromatic or white flat lighting devices, and monochromatic or white flexible lighting devices.

## Description

### Technical Field

The present invention relates to a polycyclic aromatic derivative and a highly efficient and long-lasting organic electroluminescent device with significantly improved luminous efficiency using the polycyclic aromatic derivative.

### Background Art

Organic electroluminescent devices are self-luminous devices in which electrons injected from an electron injecting electrode (cathode) recombine with holes injected from a hole injecting electrode (anode) in a light emitting layer to form excitons, which emit light while releasing energy. Such organic electroluminescent devices have the advantages of low driving voltage, high luminance, large viewing angle, and short response time and can be applied to full-color light emitting flat panel displays. Due to these advantages, organic electroluminescent devices have received attention as next-generation light sources.

The above characteristics of organic electroluminescent devices are achieved by structural optimization of organic layers of the devices and are supported by stable and efficient materials for the organic layers, such as hole injecting materials, hole transport materials, light emitting materials, electron transport materials, electron injecting materials, and electron blocking materials. However, more research still needs to be done to develop structurally optimized organic layers for organic electroluminescent devices and stable and efficient materials for organic layers of organic electroluminescent devices.

As such, there is a continued need to develop structures of organic electroluminescent devices optimized to improve their luminescent properties and new materials capable of supporting the optimized structures of organic electroluminescent devices.

### Problems to be Solved by the Invention

Accordingly, the present invention is intended to provide an organic compound that is employed in an organic layer of an organic electroluminescent device to achieve high efficiency and long lifetime of the device. The present invention is also intended to provide an organic electroluminescent device including the organic compound.

### Means for Solving the Problems

One aspect of the present invention provides an organic compound represented by Formula I or II:

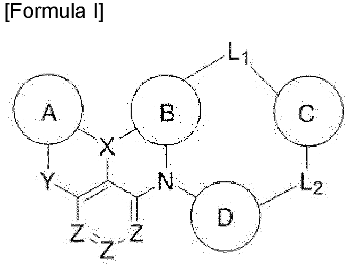

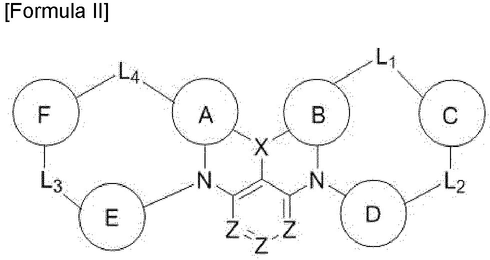

The structures of Formulae I and II, specific compounds that can be represented by Formulae I and II, and definitions of the rings A to F, X, Y, L₁ to L₄, and Z are described below.

The present invention also provides an organic electroluminescent device including a first electrode, a second electrode opposite to the first electrode, and one or more organic layers interposed between the first and second electrodes wherein one of the organic layers includes at least one of the specific polycyclic aromatic compounds that can be represented by Formula I or II.

### Effects of the Invention

The polycyclic aromatic derivative of the present invention can be employed in an organic layer of an organic electroluminescent device to achieve high efficiency and long lifetime of the device.

The present invention will now be described in more detail.

The present invention is directed to a polycyclic aromatic derivative for use in an organic electroluminescent device, represented by Formula I or II: 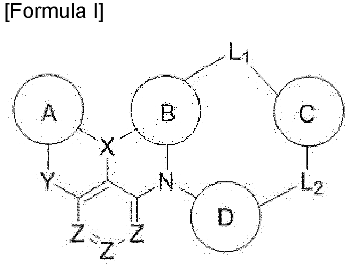 wherein the rings A to D are the same as or different from each other and are each independently a substituted or unsubstituted C₆-C₅₀ monocyclic or polycyclic aromatic hydrocarbon ring or a substituted or unsubstituted C₂-C₅₀ monocyclic or polycyclic aromatic heterocyclic ring, X is selected from B, P, P=O, P=S, and Al, L₁ and L₂ are the same as or different from each other and are each independently a single bond or selected from 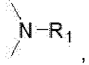 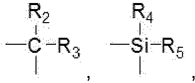 -O-, -S-, and -Se-, Y is a single bond or selected from 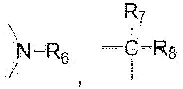 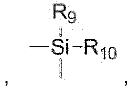 -O-, -S-, and -Se-, each Z is independently CR or N, R and R₁ to R₁₀ are the same as or different from each other and are each independently selected from hydrogen, deuterium, substituted or unsubstituted C₁-C₃₀ alkyl, substituted or unsubstituted C₁-C₃₀ alkenyl, substituted or unsubstituted C₆-C₅₀ aryl, substituted or unsubstituted C₃-C₃₀ cycloalkyl, substituted or unsubstituted C₂-C₃₀ heterocycloalkyl, substituted or unsubstituted C₂-C₅₀ heteroaryl, substituted or unsubstituted C₁-C₃₀ alkoxy, substituted or unsubstituted C₆-C₃₀ aryloxy, substituted or unsubstituted C₁-C₃₀ alkylthioxy, substituted or unsubstituted C₅-C₃₀ arylthioxy, substituted or unsubstituted amine, substituted or unsubstituted silyl, nitro, cyano, and halogen, provided that when each of L₁ and L₂ is 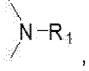 R₁ is optionally bonded to the adjacent ring C or D to form an alicyclic or aromatic monocyclic or polycyclic ring, with the proviso that each of R₆ to R₁₀ is optionally bonded to the ring A or R to form an alicyclic or aromatic monocyclic or polycyclic ring, R is optionally bonded to the ring D to form an alicyclic or aromatic monocyclic or polycyclic ring, R₂ and R₃ are optionally linked to each other to form an alicyclic or aromatic monocyclic or polycyclic ring, R₄ and R₅ are optionally linked to each other to form an alicyclic or aromatic monocyclic or polycyclic ring, R₇ and R₈ are optionally linked to each other to form an alicyclic or aromatic monocyclic or polycyclic ring, R₉ and R₁₀ are optionally linked to each other to form an alicyclic or aromatic monocyclic or polycyclic ring, a substituted or unsubstituted monocyclic or polycyclic non-aromatic ring is optionally fused to the ring A to form a fused ring, a substituted or unsubstituted monocyclic or polycyclic non-aromatic ring is optionally fused to the ring B to form a fused ring, a substituted or unsubstituted monocyclic or polycyclic non-aromatic ring is optionally fused to the ring C to form a fused ring, a substituted or unsubstituted monocyclic or polycyclic non-aromatic ring is optionally fused to the ring D to form a fused ring, and the rings A and B are optionally linked to each other, 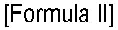 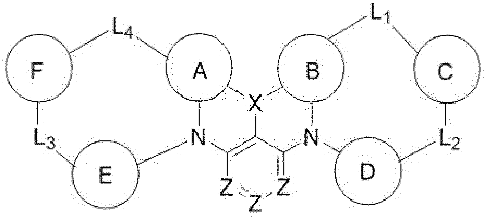 wherein the rings A to F are the same as or different from each other and are each independently a substituted or unsubstituted C₆-C₅₀ monocyclic or polycyclic aromatic hydrocarbon ring or a substituted or unsubstituted C₂-C₅₀ monocyclic or polycyclic aromatic heterocycle, X is selected from B, P, P=O, P=S, and Al, L₁ to L₄ are the same as or different from each other and are each independently a single bond or selected from 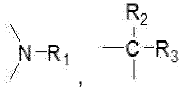 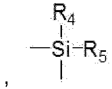 , -O-, -S-, and -Se-, each Z is independently CR or N, R and R₁ to R₅ are the same as or different from each other and are each independently selected from hydrogen, deuterium, substituted or unsubstituted C₁-C₃₀ alkyl, substituted or unsubstituted C₁-C₃₀ alkenyl, substituted or unsubstituted C₆-C₅₀ aryl, substituted or unsubstituted C₃-C₃₀ cycloalkyl, substituted or unsubstituted C₂-C₃₀ heterocycloalkyl, substituted or unsubstituted C₂-C₅₀ heteroaryl, substituted or unsubstituted C₁-C₃₀ alkoxy, substituted or unsubstituted C₆-C₃₀ aryloxy, substituted or unsubstituted C₁-C₃₀ alkylthioxy, substituted or unsubstituted C₅-C₃₀ arylthioxy, substituted or unsubstituted amine, substituted or unsubstituted silyl, nitro, cyano, and halogen, provided that when each of L₁ to L₄ is 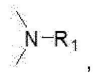 R₁ is optionally bonded to the adjacent ring C, D, E or F to form an alicyclic or aromatic monocyclic or polycyclic ring, with the proviso that R is optionally bonded to the ring D or E to form an alicyclic or aromatic monocyclic or polycyclic ring, R₂ and R₃ are optionally linked to each other to form an alicyclic or aromatic monocyclic or polycyclic ring, R₄ and R₅ are optionally linked to each other to form an alicyclic or aromatic monocyclic or polycyclic ring, a substituted or unsubstituted monocyclic or polycyclic non-aromatic ring is optionally fused to the ring A to form a fused ring, a substituted or unsubstituted monocyclic or polycyclic non-aromatic ring is optionally fused to the ring B to form a fused ring, a substituted or unsubstituted monocyclic or polycyclic non-aromatic ring is optionally fused to the ring C to form a fused ring, a substituted or unsubstituted monocyclic or polycyclic non-aromatic ring is optionally fused to the ring D to form a fused ring, a substituted or unsubstituted monocyclic or polycyclic non-aromatic ring is optionally fused to the ring E to form a fused ring, a substituted or unsubstituted monocyclic or polycyclic non-aromatic ring is optionally fused to the ring F to form a fused ring, and the rings A and B are optionally linked to each other.

The use of the polycyclic aromatic derivative makes the organic electroluminescent device highly efficient and long lasting.

According to one embodiment of the present invention, L₂ linking the rings C and D in Formula I may be provided in two and the two linkers L₂ may be the same as or different from each other and may form a ring with the rings C and D, as depicted in Formula I-1:

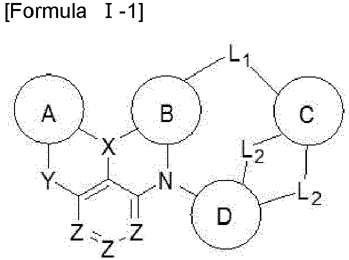

According to one embodiment of the present invention, L₂ linking the rings C and D in Formula II may be provided in two and the two linkers L₂ may be the same as or different from each other and may form a ring with the rings C and D, as depicted in Formula II-1:

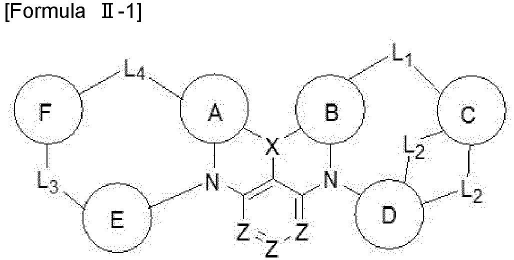

According to one embodiment of the present invention, L₃ linking the rings E and F in Formula II may be provided in two and the two linkers L₃ may be the same as or different from each other and may form a ring with the rings E and F, as depicted in Formula II-2:

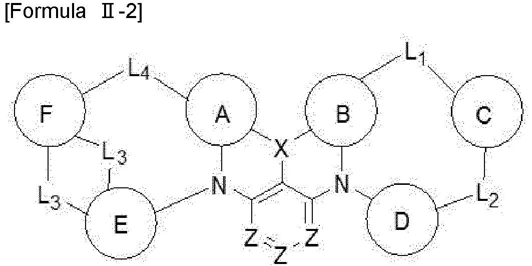

According to one embodiment of the present invention, L₂ linking the rings C and D in Formula II may be provided in two and the two linkers L₂ may form a ring with the rings C and D; and L₃ linking the rings E and F in Formula II may be provided in two and the two linkers L₃ may form a ring with the rings E and F, as depicted in Formula II-3:

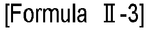

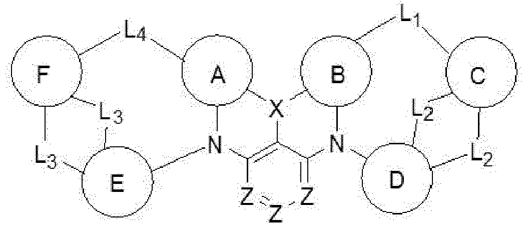

Specific structures of Formulae I and II and modified structures of Formulae I and II including additional rings can be found in the specific compounds that are exemplified below.

As used herein, the term "substituted" in the definition of the rings A to F, R, and R₁ to R₁₀ indicates substitution with one or more substituents selected from deuterium, cyano, halogen, hydroxyl, nitro, alkyl, haloalkyl, cycloalkyl, alkenyl, alkynyl, heteroalkyl, aryl, arylalkyl, alkylaryl, heteroaryl, heteroarylalkyl, alkoxy, amine, silyl, aryloxy, and mixed aliphatic-aromatic cyclic groups, or a combination thereof. As used herein, the term "unsubstituted" in the same definition indicates having no substituent.

In the "substituted or unsubstituted C₁-C₁₀ alkyl", "substituted or unsubstituted C₆-C₅₀ aryl", etc., the number of carbon atoms in the alkyl or aryl group indicates the number of carbon atoms constituting the unsubstituted alkyl or aryl moiety without considering the number of carbon atoms in the substituent(s). For example, a phenyl group substituted with a butyl group at the para-position corresponds to a C₆ aryl group substituted with a C₄ butyl group.

As used herein, the expression "form a ring with an adjacent substituent" means that the corresponding substituent combines with an adjacent substituent to form a substituted or unsubstituted alicyclic or aromatic ring and the term "adjacent substituent" may mean a substituent on an atom directly attached to an atom substituted with the corresponding substituent, a substituent disposed sterically closest to the corresponding substituent or another substituent on an atom substituted with the corresponding substituent. For example, two substituents substituted at the ortho position of a benzene ring or two substituents on the same carbon in an aliphatic ring may be considered "adjacent" to each other.

The alkyl groups may be straight or branched and specific examples thereof include, but are not limited to, methyl, ethyl, propyl, n-propyl, isopropyl, butyl, n-butyl, isobutyl, tert-butyl, sec-butyl, 1-methylbutyl, 1-ethylbutyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethylpropyl, 1,1-dimethylpropyl, isohexyl, 2-methylpentyl, 4-methylhexyl, and 5-methylhexyl groups.

The alkenyl group is intended to include straight and branched ones and may be optionally substituted with one or more other substituents. The alkenyl group may be specifically a vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, stilbenyl or styrenyl group but is not limited thereto.

The alkynyl group is intended to include straight and branched ones and may be optionally substituted with one or more other substituents. The alkynyl group may be, for example, ethynyl or 2-propynyl but is not limited thereto.

The cycloalkyl group is intended to include monocyclic and polycyclic ones and may be optionally substituted with one or more other substituents. As used herein, the term "polycyclic" means that the cycloalkyl group may be directly attached or fused to one or more other cyclic groups. The other cyclic groups may be cycloalkyl groups and other examples thereof include heterocycloalkyl, aryl, and heteroaryl groups. The cycloalkyl group may be specifically a cyclopropyl, cyclobutyl, cyclopentyl, adamantyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl or cyclooctyl group but is not limited thereto.

The heterocycloalkyl group is intended to include monocyclic and polycyclic ones interrupted by a heteroatom such as O, S, Se, N or Si and may be optionally substituted with one or more other substituents. As used herein, the term "polycyclic" means that the heterocycloalkyl group may be directly attached or fused to one or more other cyclic groups. The other cyclic groups may be heterocycloalkyl groups and other examples thereof include cycloalkyl, aryl, and heteroaryl groups.

The aromatic hydrocarbon rings or aryl groups may be monocyclic or polycyclic ones. Examples of the monocyclic aryl groups include, but are not limited to, phenyl, biphenyl, terphenyl, and stilbenyl groups. Examples of the polycyclic aryl groups include naphthyl, anthracenyl, phenanthrenyl, pyrenyl, perylenyl, tetracenyl, chrysenyl, fluorenyl, acenaphathcenyl, triphenylene, and fluoranthrene groups but the scope of the present invention is not limited thereto.

The aromatic heterocyclic rings or heteroaryl groups refer to aromatic groups interrupted by one or more heteroatoms. Examples of the aromatic heterocyclic rings or heteroaryl groups include, but are not limited to, thiophene, furan, pyrrole, imidazole, thiazole, oxazole, oxadiazole, triazole, pyridyl, bipyridyl, pyrimidyl, triazine, triazole, acridyl, pyridazine, pyrazinyl, quinolinyl, quinazoline, quinoxalinyl, phthalazinyl, pyridopyrimidinyl, pyridopyrazinyl, pyrazinopyrazinyl, isoquinoline, indole, carbazole, benzoxazole, benzimidazole, benzothiazole, benzocarbazole, benzothiophene, dibenzothiophene, benzofuranyl, dibenzofuranyl, phenanthroline, thiazolyl, isoxazolyl, oxadiazolyl, thiadiazolyl, benzothiazolyl, and phenothiazinyl groups.

The aliphatic hydrocarbon rings refer to non-aromatic rings consisting only of carbon and hydrogen atoms. The aliphatic hydrocarbon ring is intended to include monocyclic and polycyclic ones and may be optionally substituted with one or more other substituents. As used herein, the term "polycyclic" means that the aliphatic hydrocarbon ring may be directly attached or fused to one or more other cyclic groups. The other cyclic groups may be aliphatic hydrocarbon rings and other examples thereof include aliphatic heterocyclic, aryl, and heteroaryl groups. Specific examples of the aliphatic hydrocarbon rings include, but are not limited to, cycloalkyl groups such as cyclopropyl, cyclobutyl, cyclopentyl, adamantyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, and cyclooctyl, cycloalkanes such as cyclohexane and cyclopentane, and cycloalkenes such as cyclohexene and cyclopentene.

The aliphatic heterocyclic rings refer to aliphatic rings interrupted by one or more heteroatoms such as O, S, Se, N, and Si. The aliphatic heterocyclic ring is intended to include monocyclic or polycyclic ones and may be optionally substituted with one or more other substituents. As used herein, the term "polycyclic" means that the aliphatic heterocyclic ring such as heterocycloalkyl, heterocycloalkane or heterocycloalkene may be directly attached or fused to one or more other cyclic groups. The other cyclic groups may be aliphatic heterocyclic rings and other examples thereof include aliphatic hydrocarbon rings, aryl groups, and heteroaryl groups.

The mixed aliphatic-aromatic ring refers to a ring in which at least one aliphatic ring and at least one aromatic ring are linked or fused together and which is overall non-aromatic. The mixed aliphatic-aromatic polycyclic ring may contain one or more heteroatoms selected from N, O, P, and S other than carbon atoms (C).

The alkoxy group may be specifically a methoxy, ethoxy, propoxy, isobutyloxy, sec-butyloxy, pentyloxy, iso-amyloxy or hexyloxy group but is not limited thereto.

The silyl groups may be, for example, -SiH₃, alkylsilyl, arylsilyl, alkylarylsilyl, and arylheteroarylsilyl. Specific examples of the silyl groups include trimethylsilyl, triethylsilyl, triphenylsilyl, trimethoxysilyl, dimethoxyphenylsilyl, diphenylmethylsilyl, diphenylvinylsilyl, methylcyclobutylsilyl, and dimethylfurylsilyl.

The amine groups may be, for example, -NH₂, alkylamine groups, arylamine groups, and arylheteroarylamine groups. The arylamine groups refer to aryl-substituted amine groups, the alkylamine groups refer to alkyl-substituted amine groups, and the arylheteroarylamine groups refer to aryl- and heteroaryl-substituted amine groups. Examples of the arylamine groups include substituted or unsubstituted monoarylamine groups, substituted or unsubstituted diarylamine groups, and substituted or unsubstituted triarylamine groups. The aryl and heteroaryl moieties in the arylamine and arylheteroarylamine groups may be monocyclic or polycyclic ones. The arylamine groups may include two or more aryl moieties and the arylheteroarylamine groups may include two or more heteroaryl moieties. In this case, the aryl moieties may be monocyclic or polycyclic aryl moieties or may consist of both monocyclic and polycyclic aryl moieties. The heteroaryl moieties may be monocyclic or polycyclic heteroaryl moieties or may consist of both monocyclic and polycyclic heteroaryl moieties. The aryl and heteroaryl moieties in the arylamine and arylheteroarylamine groups may be selected from those exemplified above.

The aryl moieties in the aryloxy group and the arylthioxy group are the same as those described above for the aryl groups. Specific examples of the aryloxy groups include, but are not limited to, phenoxy, p-tolyloxy, m-tolyloxy, 3,5-dimethylphenoxy, 2,4,6-trimethylphenoxy, p-tert-butylphenoxy, 3-biphenyloxy, 4-biphenyloxy, 1-naphthyloxy, 2-naphthyloxy, 4-methyl-1-naphthyloxy, 5-methyl-2-naphthyloxy, 1-anthryloxy, 2-anthryloxy, 9-anthryloxy, 1-phenanthryloxy, 3-phenanthryloxy, and 9-phenanthryloxy groups. The arylthioxy group may be, for example, a phenylthioxy, 2-methylphenylthioxy or 4-tert-butylphenylthioxy group but is not limited thereto.

The halogen group may be, for example, fluorine, chlorine, bromine or iodine.

More specifically, the polycyclic aromatic derivative represented by Formula I or II according to the present invention may be selected from, but not limited to, the following compounds 1 to 210:

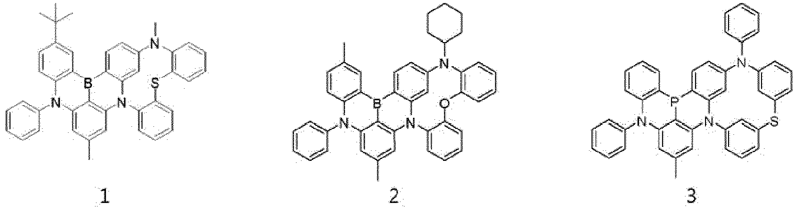

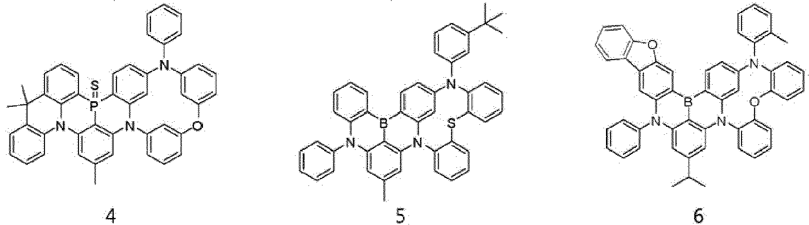

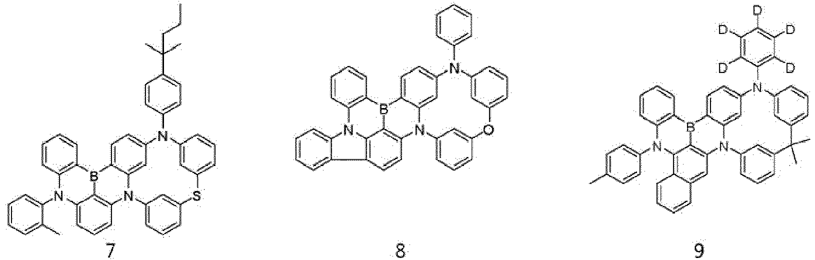

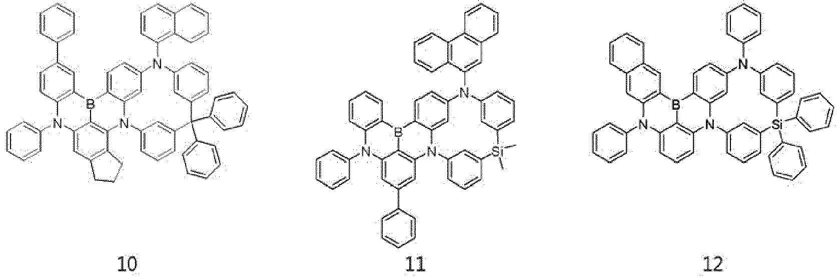

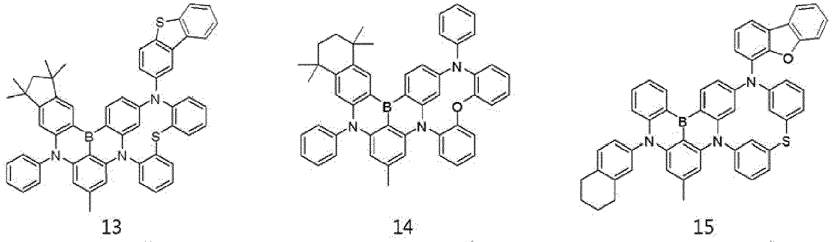

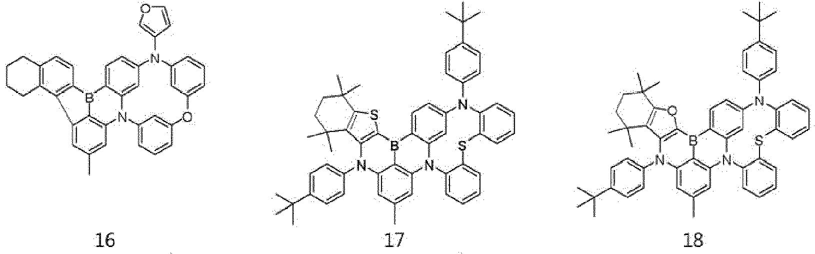

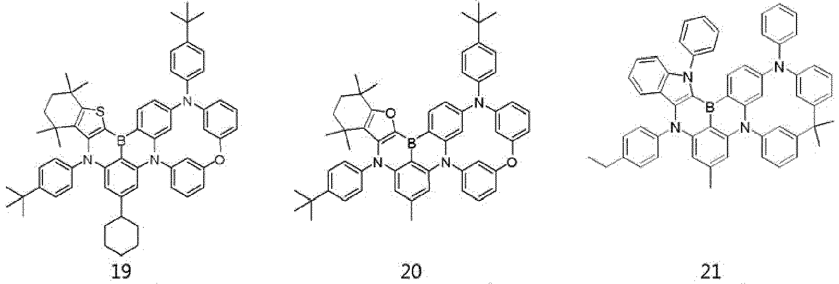

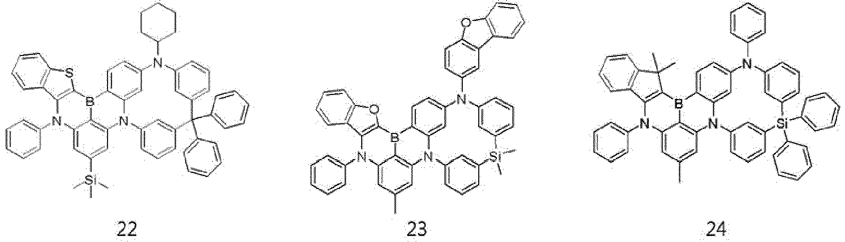

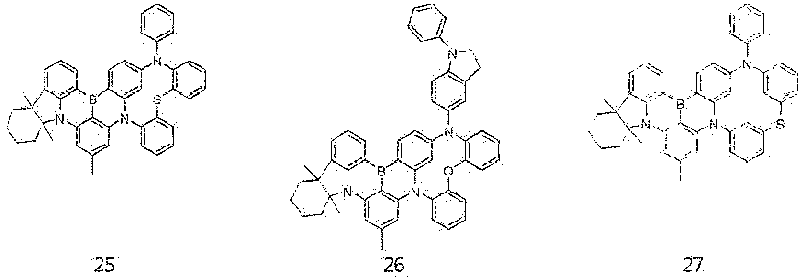

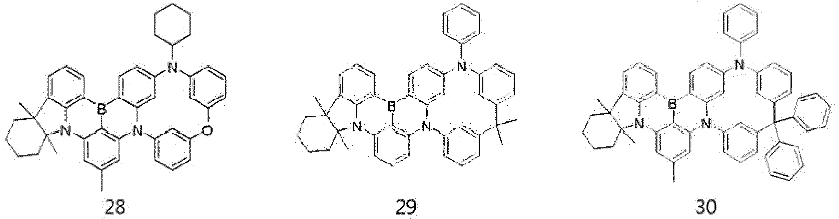

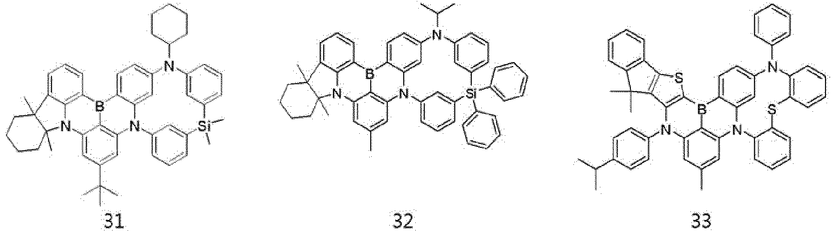

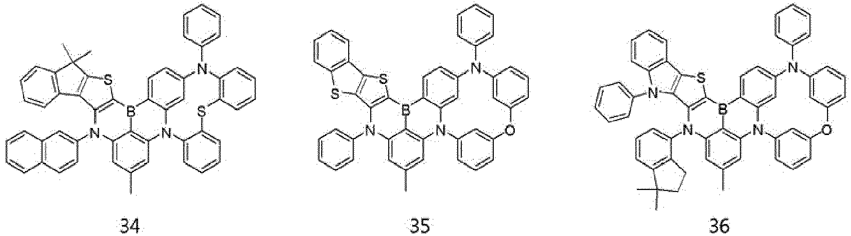

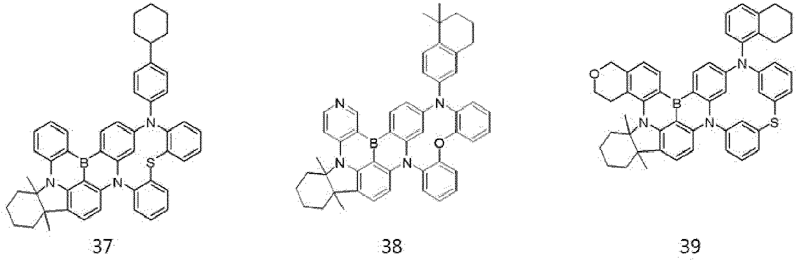

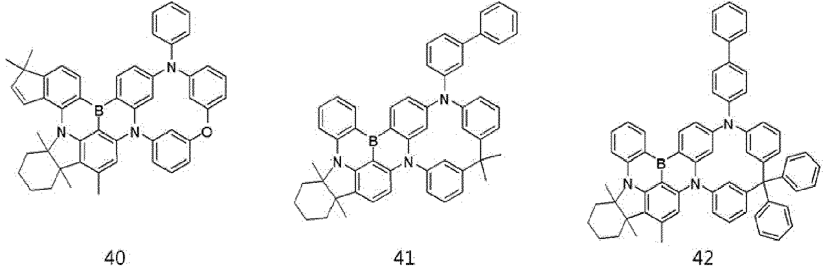

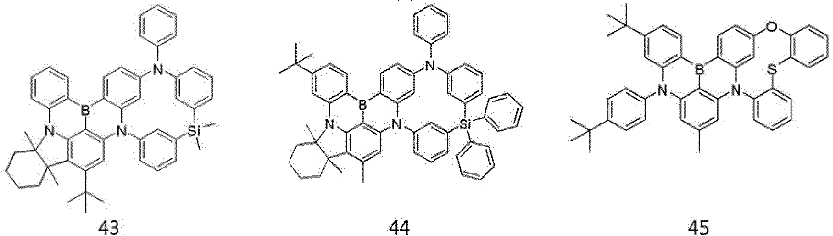

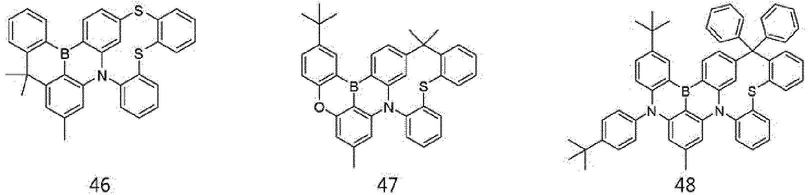

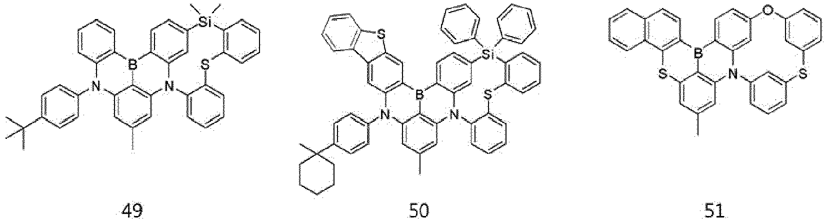

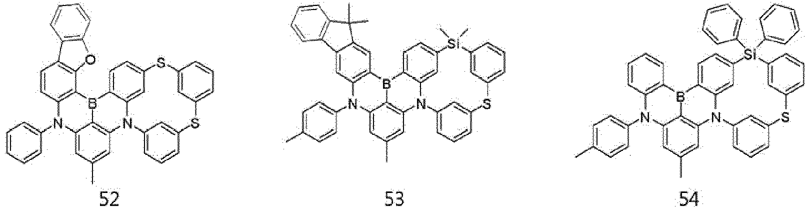

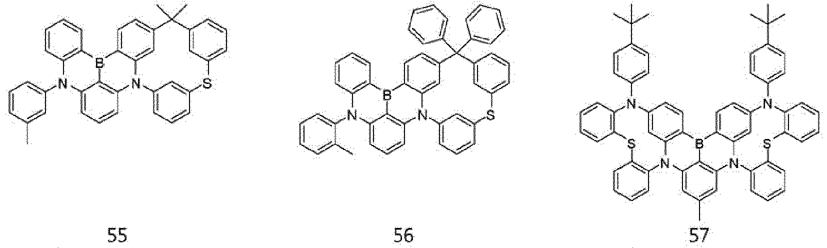

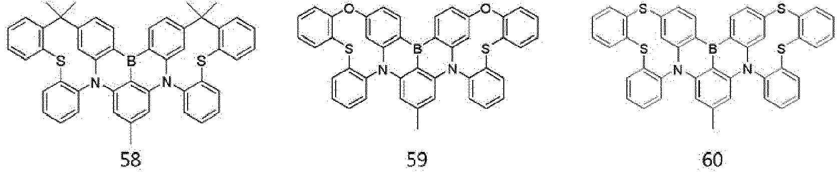

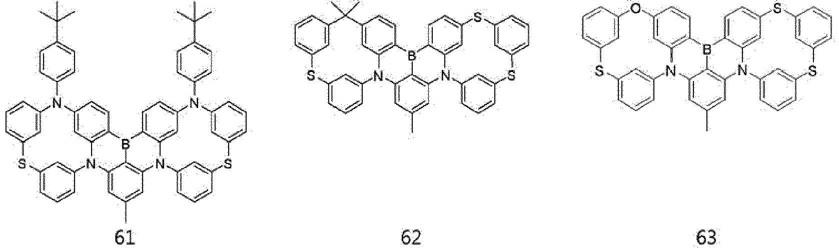

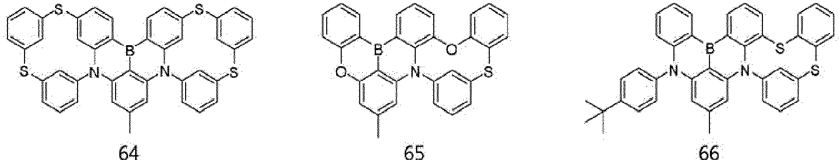

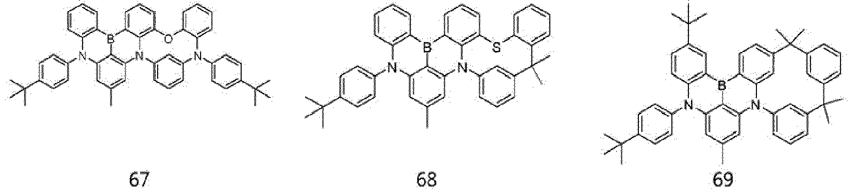

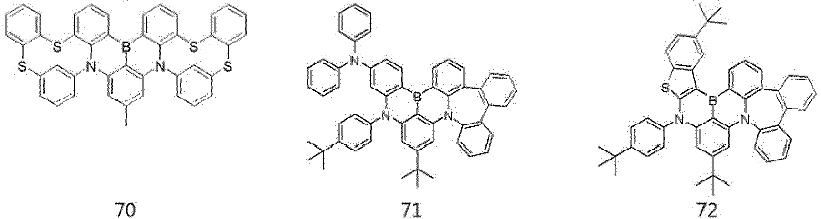

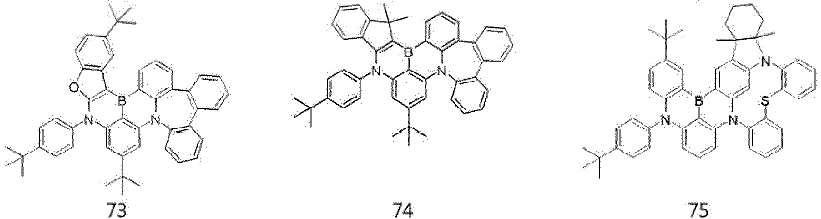

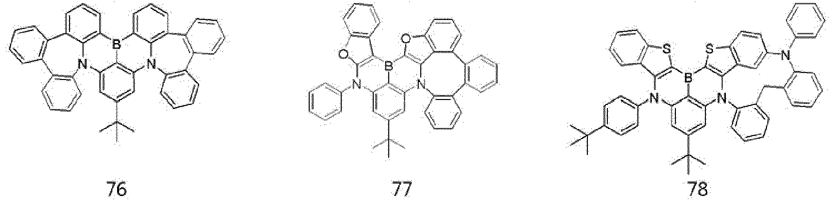

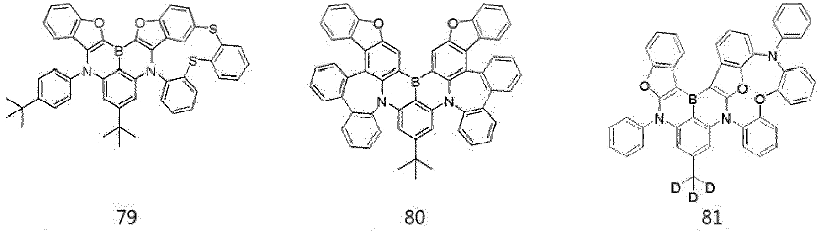

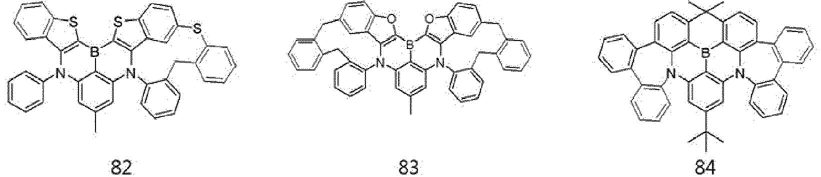

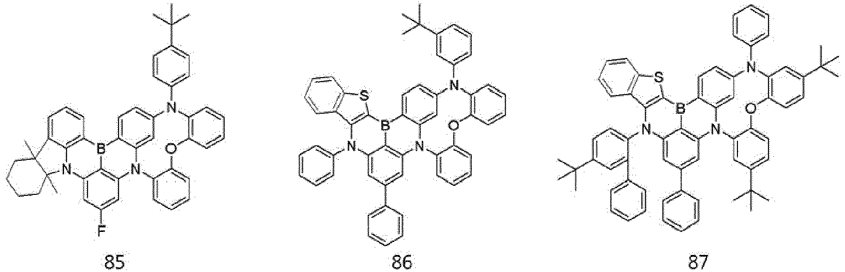

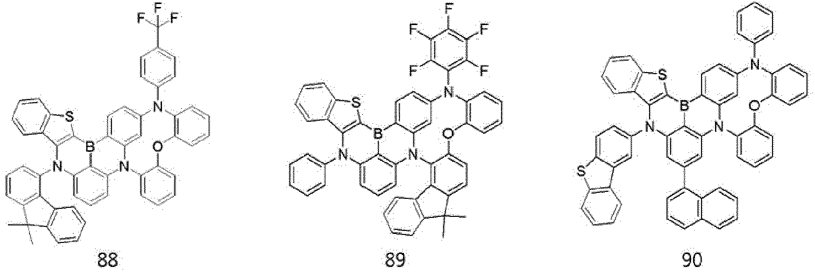

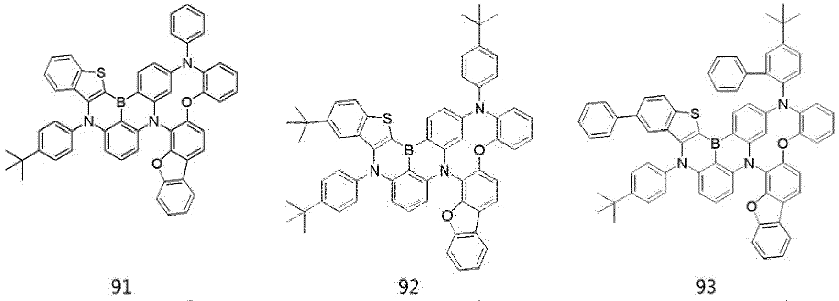

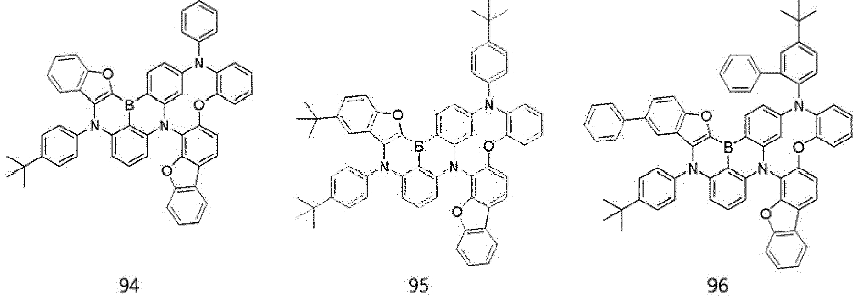

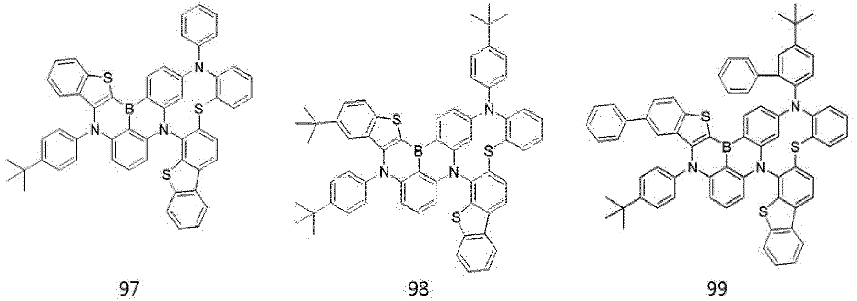

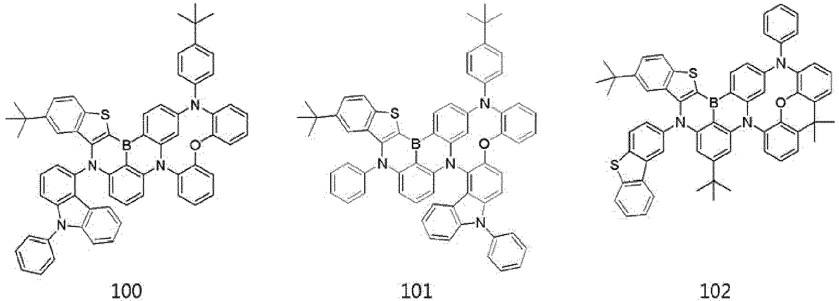

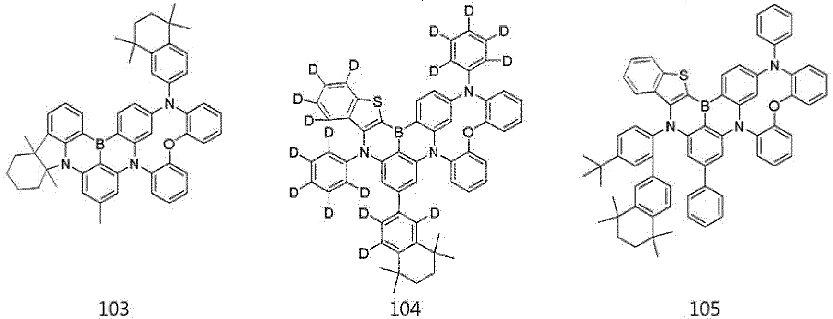

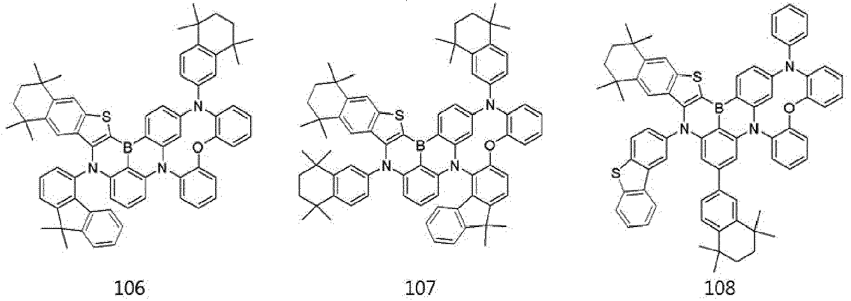

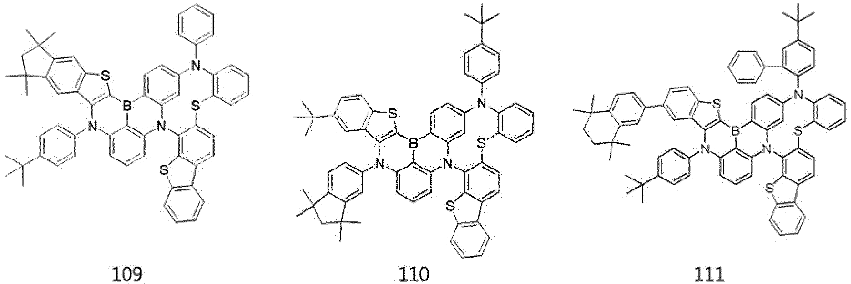

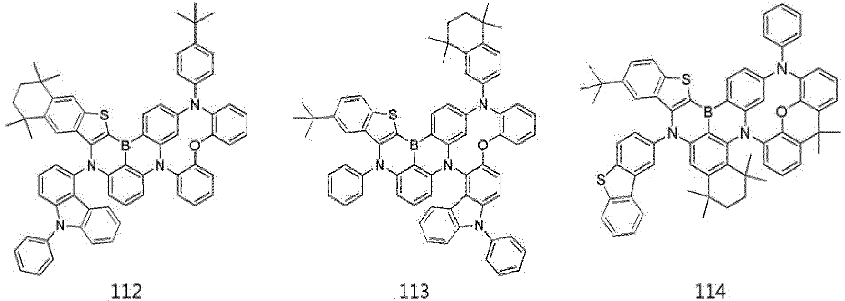

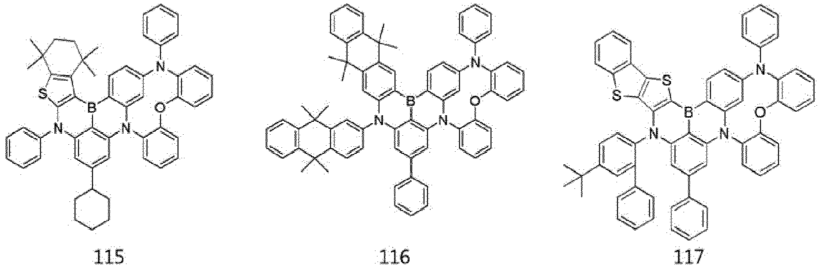

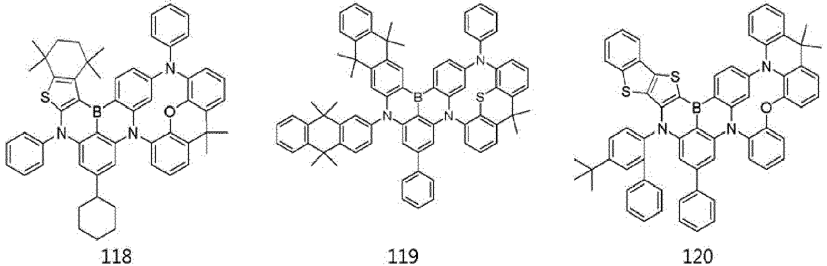

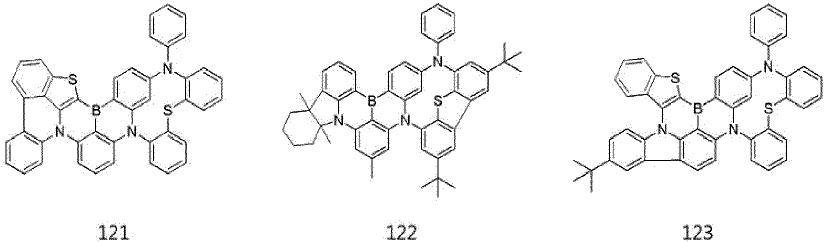

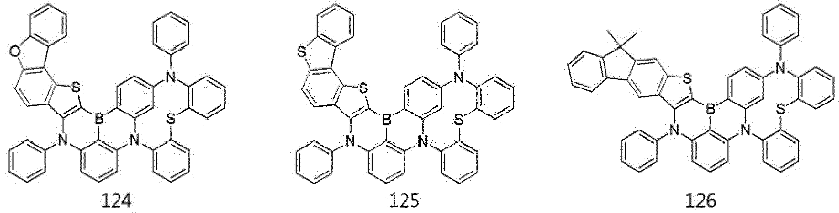

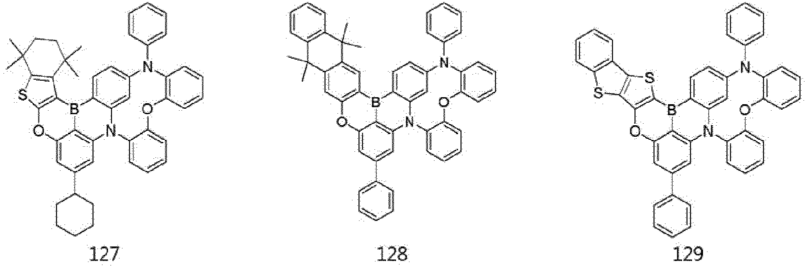

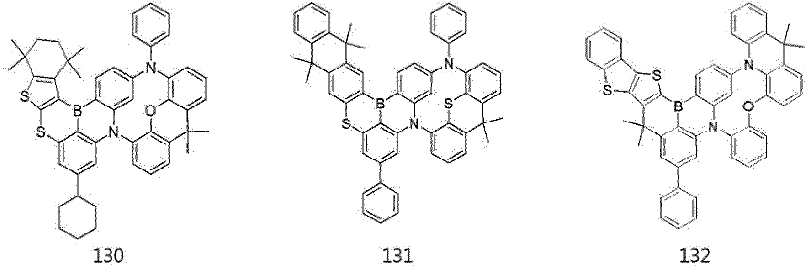

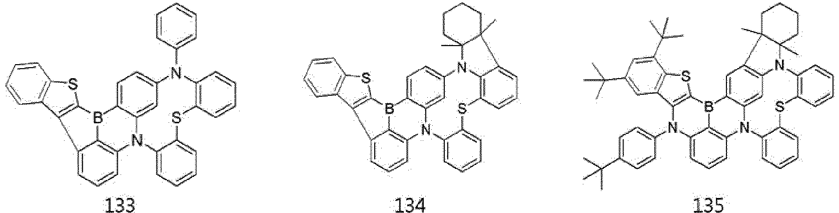

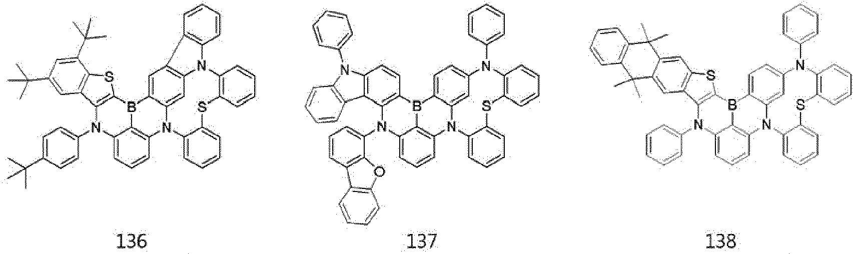

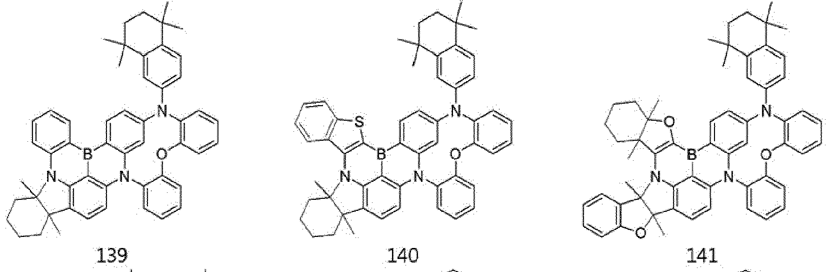

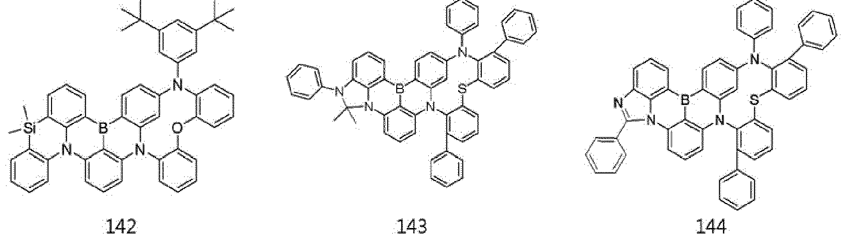

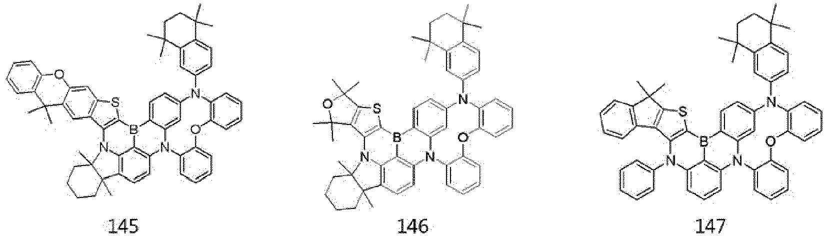

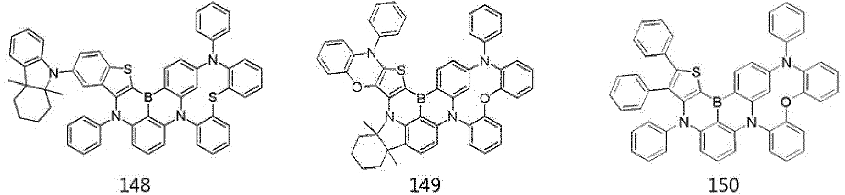

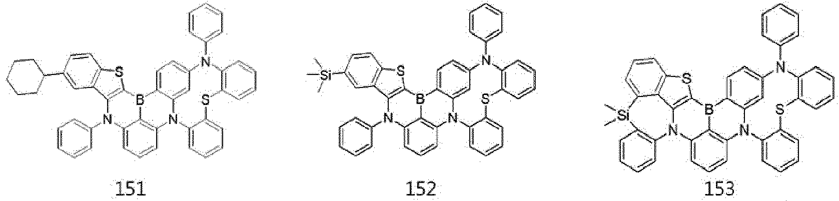

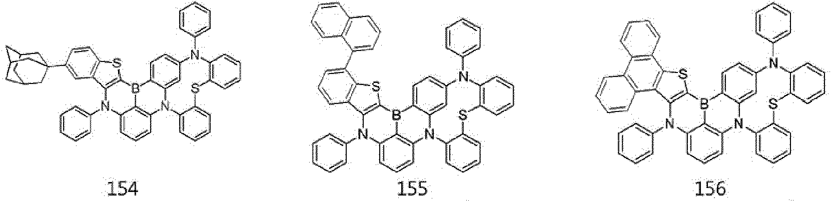

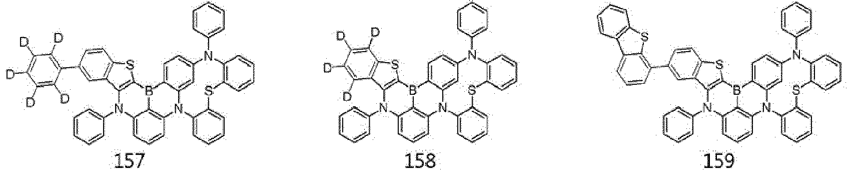

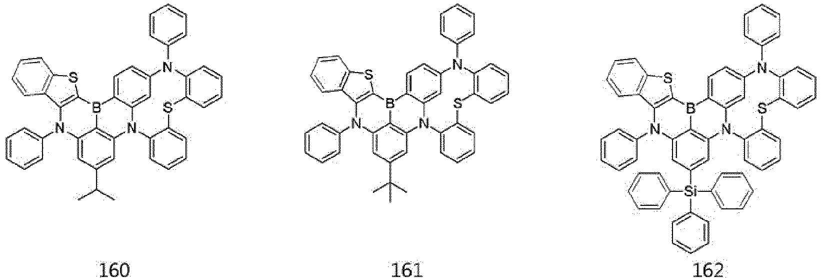

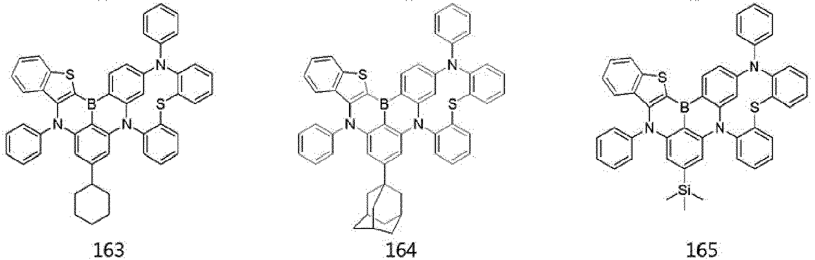

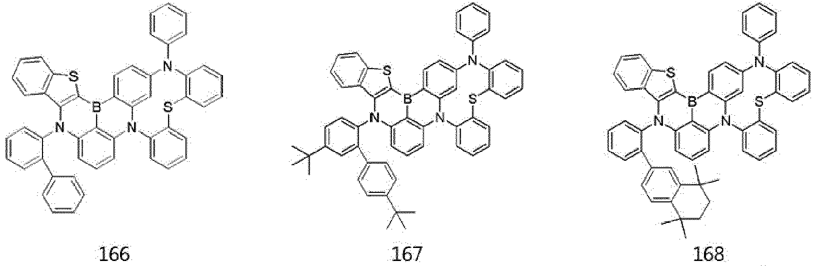

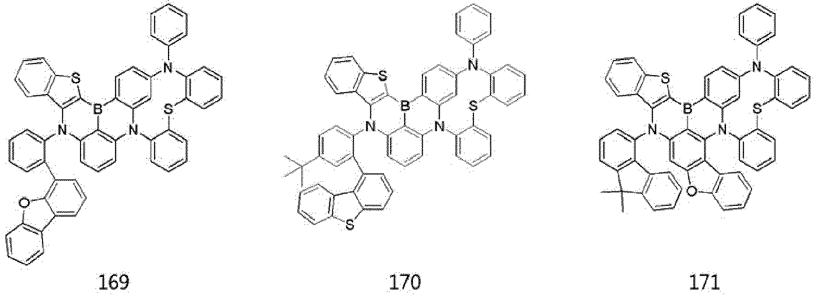

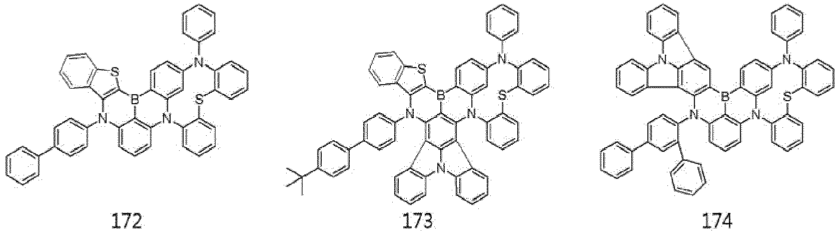

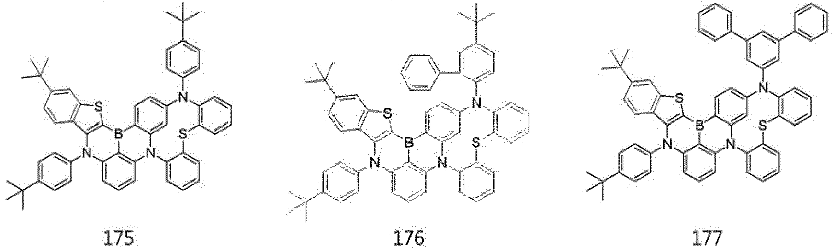

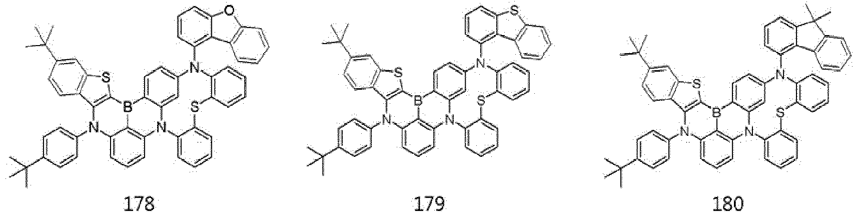

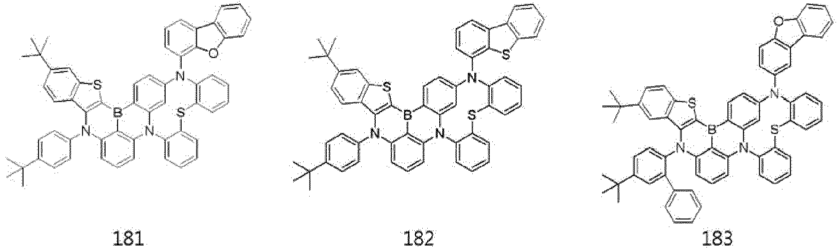

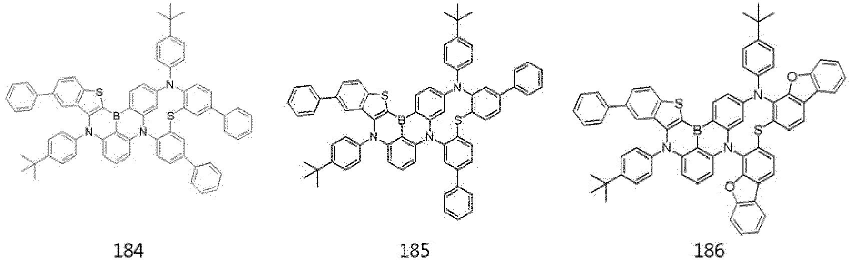

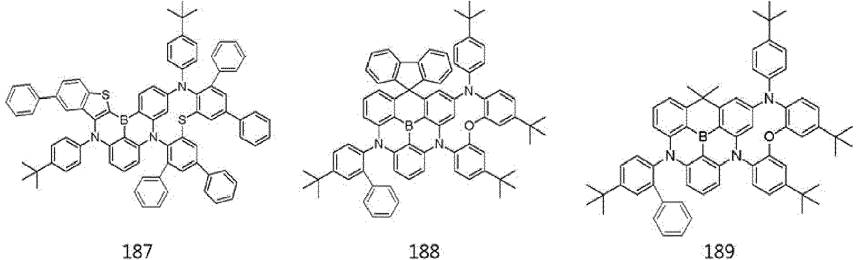

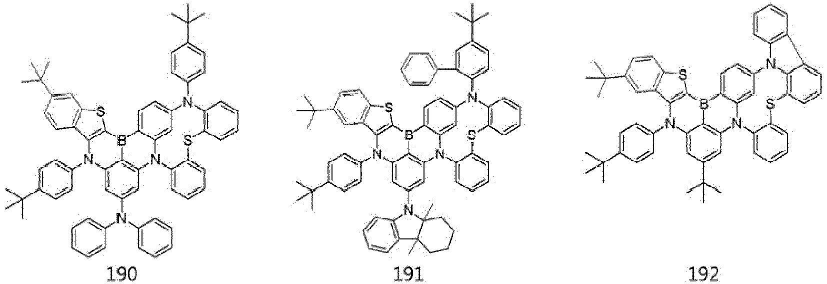

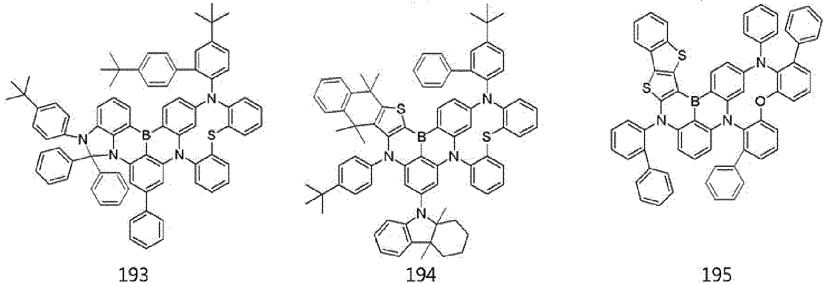

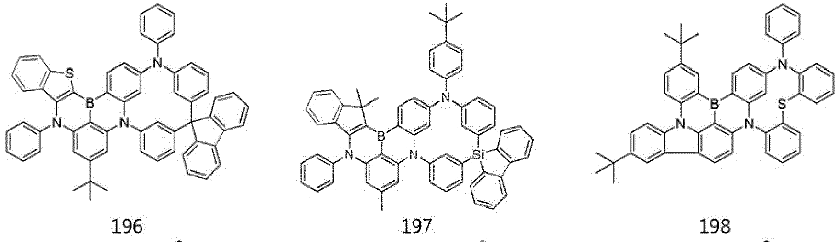

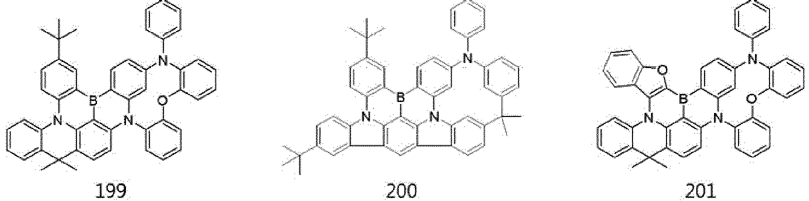

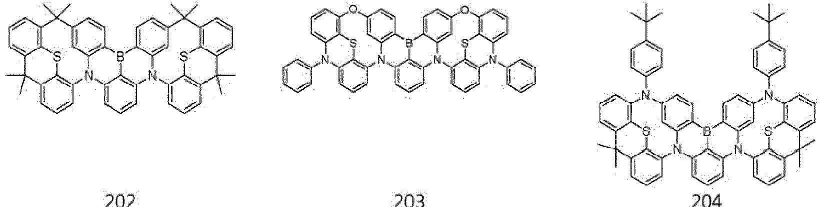

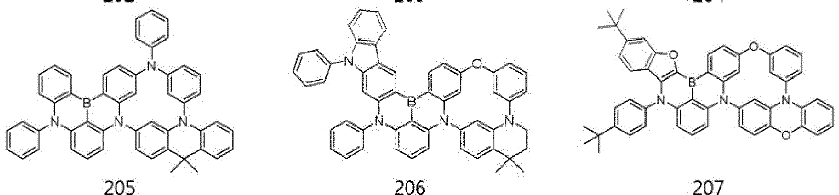

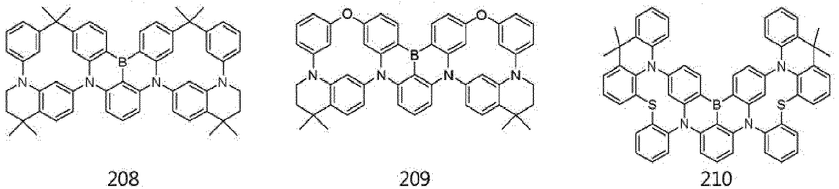

The specific substituents in Formula I or II can be clearly seen from the structures of the compounds 1 to 210 but are not intended to limit the scope of the compound represented by Formula I or II.

As can be seen from the above specific compounds, the polycyclic aromatic derivative of the present invention contains B, P, P=O, P=S or Al and has a polycyclic aromatic structure. The introduction of substituents into the polycyclic aromatic structure enables the synthesis of organic materials with inherent characteristics of the substituents. For example, the substituents are designed for use in materials for hole injecting layers, hole transport layers, light emitting layers, electron transport layers, electron injecting layers, electron blocking layers, and hole blocking layers of organic electroluminescent devices. This introduction meets the requirements of materials for the organic layers, making the organic electroluminescent devices highly efficient.

A further aspect of the present invention is directed to an organic electroluminescent device including a first electrode, a second electrode, and one or more organic layers interposed between the first and second electrodes wherein one of the organic layers includes at least one of the organic compounds that can be represented by Formula I or II.

That is, according to one embodiment of the present invention, the organic electroluminescent device has a structure in which one or more organic layers are arranged between a first electrode and a second electrode. The organic electroluminescent device of the present invention may be fabricated by a suitable method known in the art using suitable materials known in the art, except that the organic compound of Formula I or II is used to form the corresponding organic layer.

The organic layers of the organic electroluminescent device according to the present invention may form a monolayer structure. Alternatively, the organic layers may have a multilayer stack structure. For example, the organic layers may have a structure including a hole injecting layer, a hole transport layer, a hole blocking layer, a light emitting layer, an electron blocking layer, an electron transport layer, and an electron injecting layer but are not limited to this structure. The number of the organic layers is not limited and may be increased or decreased. Preferred structures of the organic layers of the organic electroluminescent device according to the present invention will be explained in more detail in the Examples section that follows.

The organic electroluminescent device of the present invention includes an anode, a hole transport layer, a light emitting layer, an electron transport layer, and a cathode. The organic electroluminescent device of the present invention may optionally further include a hole injecting layer between the anode and the hole transport layer and an electron injecting layer between the electron transport layer and the cathode. If necessary, the organic electroluminescent device of the present invention may further include one or two intermediate layers such as a hole blocking layer or an electron blocking layer.

According to one embodiment of the present invention, one of the organic layers interposed between the first and second electrodes may be a light emitting layer composed of a host and the compound represented by Formula I or II as a dopant. The content of the dopant in the light emitting layer is typically in the range of about 0.01 to about 20 parts by weight, based on about 100 parts by weight of the host but is not limited to this range.

According to one embodiment of the present invention, the host may be an anthracene derivative represented by Formula C: 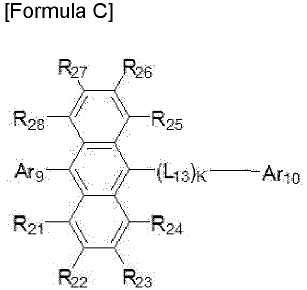 wherein R₂₁ to R₂₈ are identical to or different from each other and are as defined for R in Formula I or II, Ar₉ and Ar₁₀ are identical to or different from each other and are each independently selected from hydrogen, deuterium, substituted or unsubstituted C₁-C₃₀ alkyl, substituted or unsubstituted C₆-C₅₀ aryl, substituted or unsubstituted C₂-C₃₀ alkenyl, substituted or unsubstituted C₂-C₂₀ alkynyl, substituted or unsubstituted C₃-C₃₀ cycloalkyl, substituted or unsubstituted C₅-C₃₀ cycloalkenyl, substituted or unsubstituted C₂-C₅₀ heteroaryl, substituted or unsubstituted C₂-C₃₀ heterocycloalkyl, substituted or unsubstituted C₁-C₃₀ alkoxy, substituted or unsubstituted C₆-C₃₀ aryloxy, substituted or unsubstituted C₁-C₃₀ alkylthioxy, substituted or unsubstituted C₆-C₃₀ arylthioxy, halogen, substituted or unsubstituted amine, and substituted or unsubstituted silyl, L₁₃ is a single bond or is selected from substituted or unsubstituted C₆-C₂₀ arylene and substituted or unsubstituted C₂-C₂₀ heteroarylene, and k is an integer from 1 to 3, provided that when k is 2 or more, the linkers L₁₃ are identical to or different from each other.

According to one embodiment of the present invention, Ar₉ in Formula C may be represented by Formula C-1: 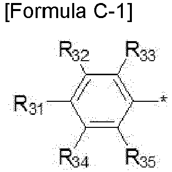 wherein R₃₁ to R₃₅ are identical to or different from each other and are as defined for R in Formula I or II and each of R₃₁ to R₃₅ is optionally bonded to an adjacent substituent to form a saturated or unsaturated ring.

According to one embodiment of the present invention, the host represented by Formula C may be selected from, but not limited to, the compounds of Formulae C1 to C66:

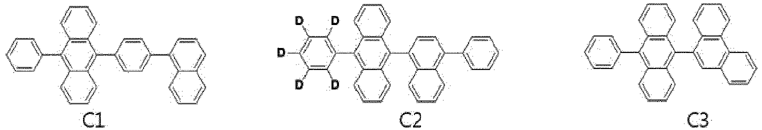

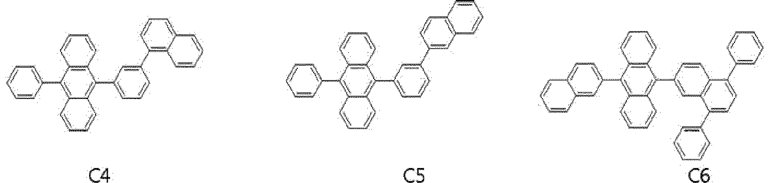

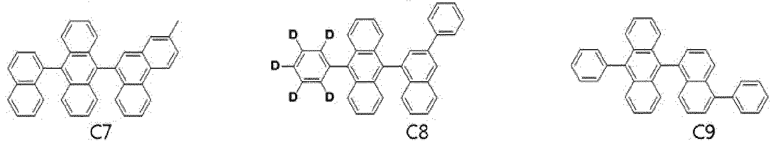

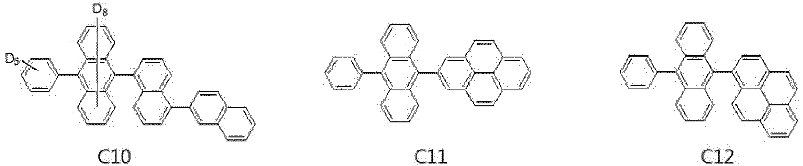

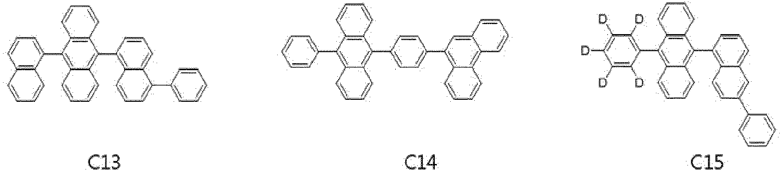

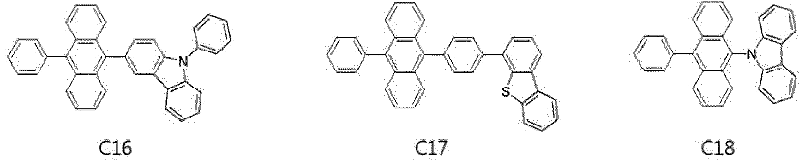

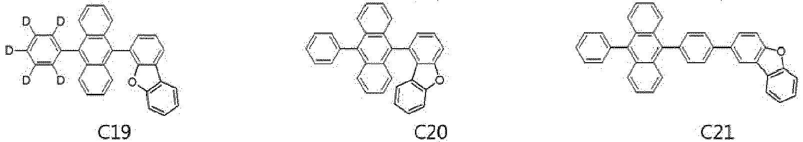

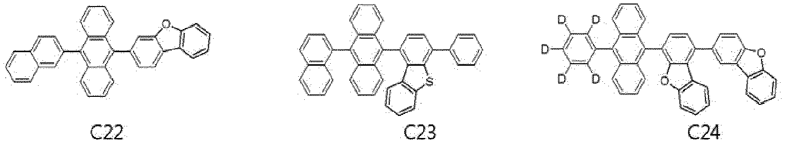

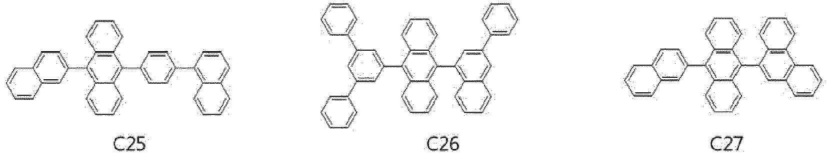

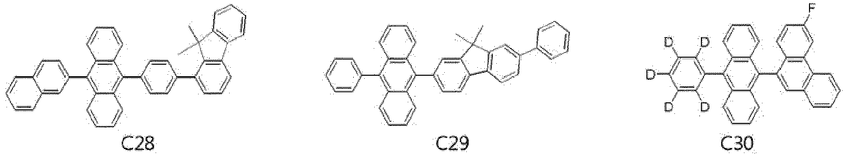

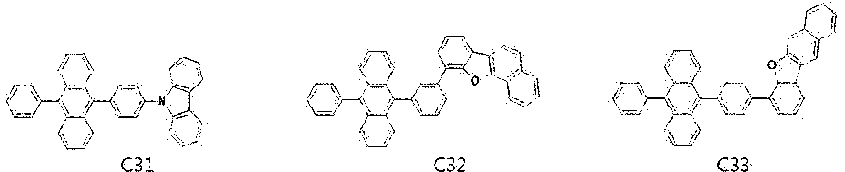

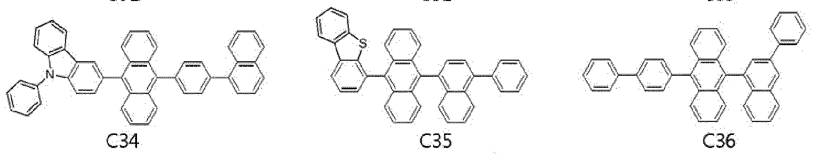

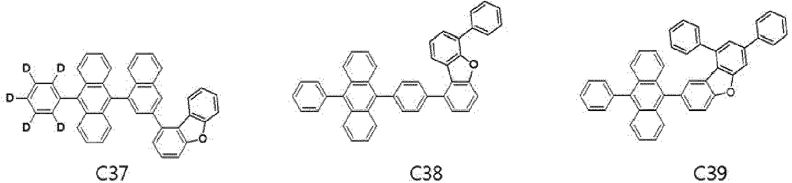

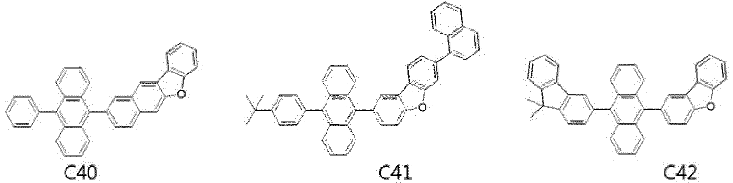

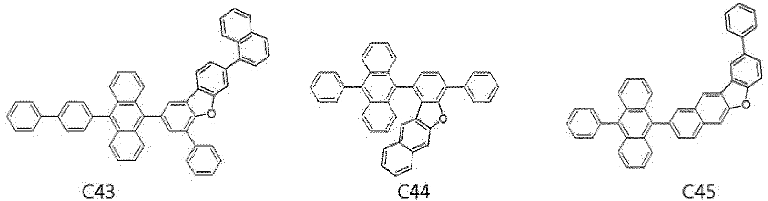

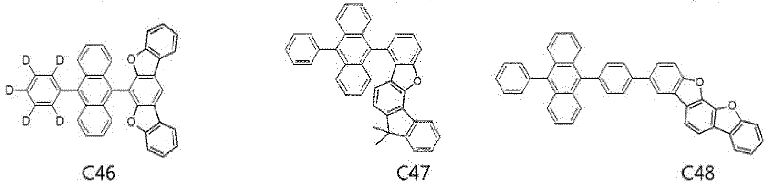

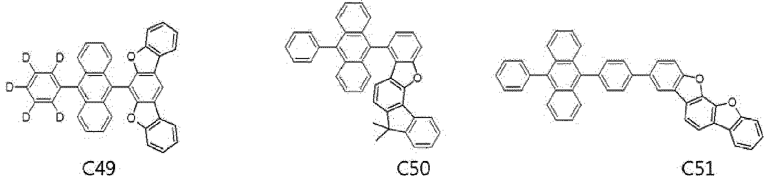

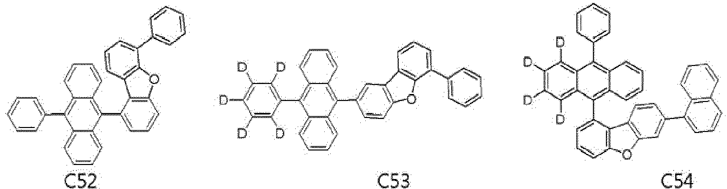

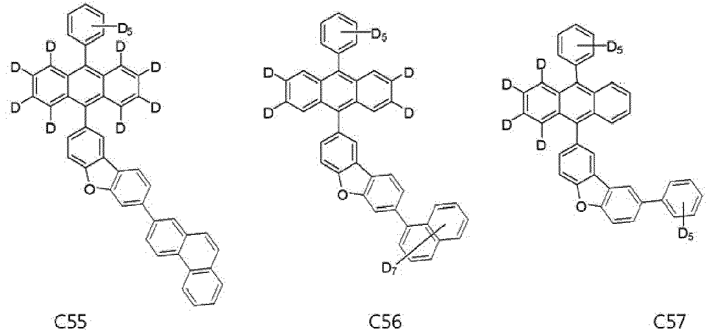

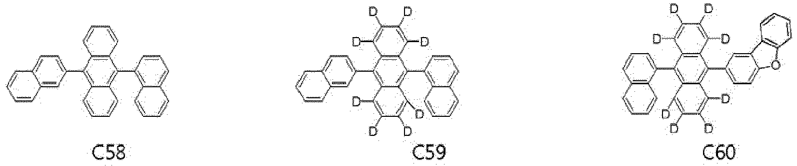

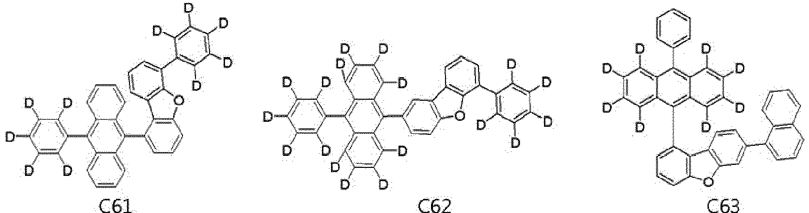

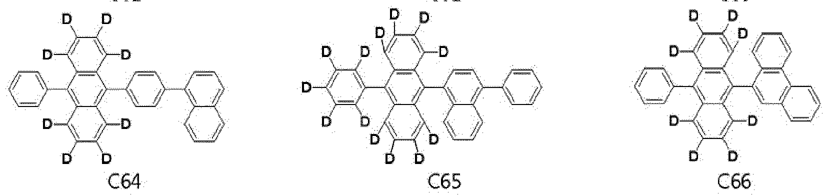

A specific structure of the organic electroluminescent device according to the present invention, a method for fabricating the device, and materials for the organic layers will be described below.

First, an anode material is coated on a substrate to form an anode. The substrate may be any of those used in general electroluminescent devices. The substrate is preferably an organic substrate or a transparent plastic substrate that is excellent in transparency, surface smoothness, ease of handling, and waterproofness. A highly transparent and conductive metal oxide such as indium tin oxide (ITO), indium zinc oxide (IZO), tin oxide (SnO₂) or zinc oxide (ZnO) is used as the anode material.

A hole injecting material is coated on the anode by vacuum thermal evaporation or spin coating to form a hole injecting layer. Then, a hole transport material is coated on the hole injecting layer by vacuum thermal evaporation or spin coating to form a hole transport layer.

The hole injecting material is not specially limited as long as it is usually used in the art. Specific examples of such materials include 4,4',4"-tris(2-naphthylphenyl-phenylamino)triphenylamine (2-TNATA), N,N'-di(1-naphthyl)-N,N'-diphenylbenzidine (NPD), N,N'-diphenyl-N,N'-bis(3-methylphenyl)-1,1'-biphenyl-4,4'-diamine (TPD), N,N'-diphenyl-N,N'-bis(4-(phenyl-m-tolylamino)phenyl)biphenyl-4,4'-diamine (DNTPD), and 1,4,5,8,9,11-hexaazatriphenylenehexacarbonitrile (HAT-CN).

The hole transport material is not specially limited as long as it is commonly used in the art. Examples of such materials include N,N'-bis(3-methylphenyl)-N,N'-diphenyl-(1,1-biphenyl)-4,4'-diamine (TPD) and N,N'-di(naphthalen-1-yl)-N,N'-diphenylbenzidine (α-NPD).

Subsequently, a hole auxiliary layer and a light emitting layer are sequentially formed on the hole transport layer. A hole blocking layer may be optionally formed on the light emitting layer by vacuum thermal evaporation or spin coating. The hole blocking layer is formed as a thin film and blocks holes from entering a cathode through the organic light emitting layer. This role of the hole blocking layer prevents the lifetime and efficiency of the device from deteriorating. A material having a very low highest occupied molecular orbital (HOMO) energy level is used for the hole blocking layer. The hole blocking material is not particularly limited as long as it can transport electrons and has a higher ionization potential than the light emitting compound. Representative examples of suitable hole blocking materials include BAIq, BCP, and TPBI.

Examples of materials for the hole blocking layer include, but are not limited to, BAIq, BCP, Bphen, TPBI, NTAZ, BeBq₂, OXD-7, and Liq.

An electron transport layer is deposited on the hole blocking layer by vacuum thermal evaporation or spin coating, and an electron injecting layer is formed thereon. A cathode metal is deposited on the electron injecting layer by vacuum thermal evaporation to form a cathode, completing the fabrication of the organic electroluminescent device.

For example, lithium (Li), magnesium (Mg), aluminum (Al), aluminum-lithium (Al-Li), calcium (Ca), magnesium-indium (Mg-In) or magnesium-silver (Mg-Ag) may be used as the metal for the formation of the cathode. The organic electroluminescent device may be of top emission type. In this case, a transmissive material such as ITO or IZO may be used to form the cathode.

A material for the electron transport layer functions to stably transport electrons injected from the cathode. The electron transport material may be any of those known in the art and examples thereof include, but are not limited to, quinoline derivatives, particularly tris(8-quinolinolate)aluminum (Alq3), TAZ, Balq, beryllium bis(benzoquinolin-10-olate (Bebq2), and oxadiazole derivatives such as PBD, BMD, and BND.

Each of the organic layers can be formed by a monomolecular deposition or solution process. According to the monomolecular deposition process, the material for each layer is evaporated into a thin film under heat and vacuum or reduced pressure. According to the solution process, the material for each layer is mixed with a suitable solvent, and then the mixture is formed into a thin film by a suitable method such as ink-jet printing, roll-to-roll coating, screen printing, spray coating, dip coating or spin coating.

The organic electroluminescent device of the present invention can be used in a display or lighting system selected from flat panel displays, flexible displays, monochromatic flat panel lighting systems, white flat panel lighting systems, flexible monochromatic lighting systems, and flexible white lighting systems.

### Mode for Carrying out the Invention

The present invention will be explained more specifically with reference to the following examples. However, it will be obvious to those skilled in the art that these examples are in no way intended to limit the scope of the invention.

### Synthesis Example 1. Preparation of Compound A

### Synthesis Example 1-1. Synthesis of Intermediate A-1

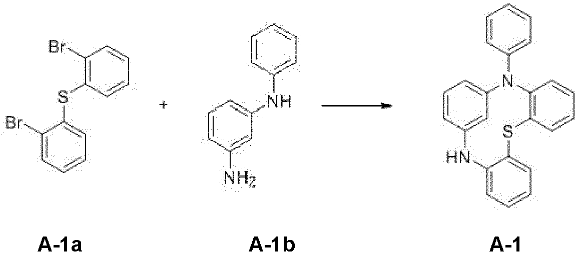

**A-1a** (50.0 g) (see Angewandte Chemie-International Edition, 2008, vol. 47, #9, p. 1726-1728 for synthesis), **A-1b** (26.8 g) (see European Journal of Medicinal Chemistry, 2017, vol. 134, p. 230-241 for synthesis), bis(tri-tert-butylphosphine)palladium(0) (2.23 g), sodium tert-butoxide (27.9 g), and toluene (500 mL) were placed in a reactor. The mixture was stirred under reflux for 24 h. The reaction mixture was cooled to room temperature and water (200 mL) was added thereto. The organic layer was extracted with ethyl acetate, concentrated under reduced pressure, and purified by silica gel column chromatography to afford **A-1** (15.1 g, yield 28.4%).
MS (ESI) calcd for Chemical Formula: C₂₄H₁₉N₂S (Pos)367.13, found 367.1

### Synthesis Example 1-2. Synthesis of Intermediate A-2

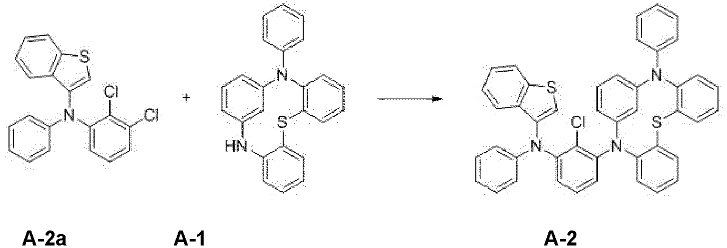

**A-2a** (12.0 g) (see US 2020 / 395 553 A1 for synthesis), **A-1** (11.9 g), bis(tri-tert-butylphosphine)palladium(0) (0.33 g), sodium tert-butoxide (6.23 g), and toluene (120 mL) were placed in a reactor. The mixture was stirred under reflux for 18 h. The reaction mixture was cooled to room temperature and water (50 mL) was added thereto. The organic layer was extracted with ethyl acetate, concentrated under reduced pressure, and purified by silica gel column chromatography to afford **A-2** (17.8 g, yield 78.4%).
MS (ESI) calcd for Chemical Formula: C₄₄H₃₁ClN₃S₂ (Pos)700.17, found 700.1

### Synthesis Example 1-3. Synthesis of Compound A

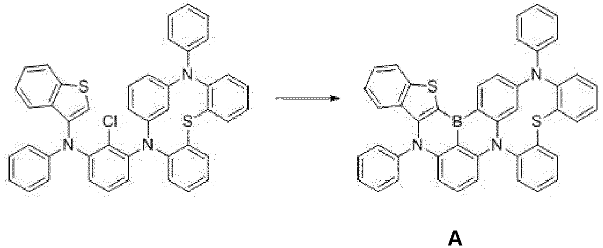

**A-2** (12.0 g) and tert-butylbenzene (120 mL) were placed in a reactor and then 1.7 M tert-butyllithium (30.2 mL) was added dropwise thereto at -60 °C. The mixture was heated to 60 °C, followed by stirring for 2 h. The reaction mixture was cooled to -60 °C and boron tribromide (3.3 mL) was added dropwise thereto. The resulting mixture was allowed to warm to room temperature, followed by stirring for 1 h. The mixture was cooled to 0 °C and N,N-diisopropylethylamine (6.0 mL) was added dropwise thereto. The temperature was raised to 120 °C, followed by stirring for 16 h. The reaction mixture was cooled to room temperature and water (50 mL) and sodium acetate (2.8 g) were added thereto. The organic layer was extracted with ethyl acetate, concentrated under reduced pressure, and purified by silica gel column chromatography to afford Compound A (2.1 g, yield 18.2%).
MS (ESI) calcd for Chemical Formula: C₄₄H₂₉BN₃S₂ (Pos)674.19, found 674.1

### Synthesis Example 2. Preparation of Compound B

### Synthesis Example 2-1. Synthesis of Compound B

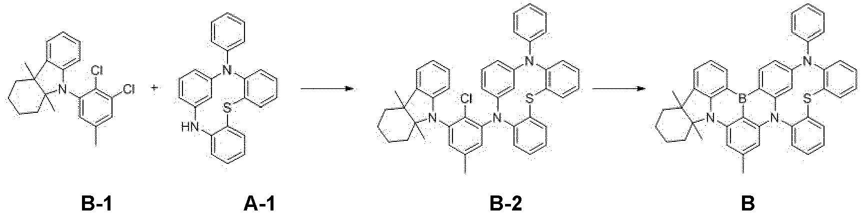

Compound B (yield 17.9%) was synthesized from Intermediate **B-2** in the same manner as in Synthesis Example 1, except that **B-1** (see Korean Patent Publication No. 2239994B1 for synthesis) was used instead of Intermediate **A-2a.**
MS (ESI) calcd for Chemical Formula: C₄₅H₃₉BN₃S (Pos)664.30, found 664.3

### Synthesis Example 3. Preparation of Compound C

### Synthesis Example 3-1. Synthesis of Compound C

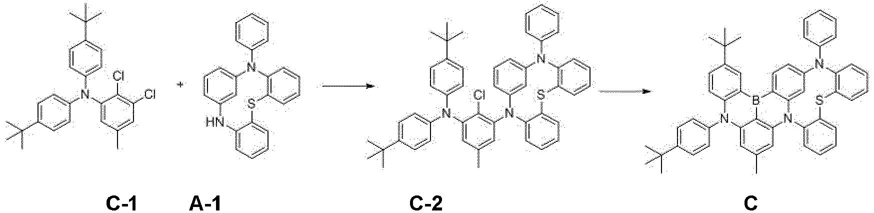

Compound **C** (yield 19.3%) was synthesized from Intermediate **C-2** in the same manner as in Synthesis Example 1, except that **C-1** (see Korean Patent Publication No. 2020-009090158A for synthesis) was used instead of Intermediate **A-2a.**
MS (ESI) calcd for Chemical Formula: C₅₁H₄₇BN₃S (Pos)744.36, found 744.3

### Synthesis Example 4. Preparation of Compound D

### Synthesis Example 4-1. Synthesis of Compound D

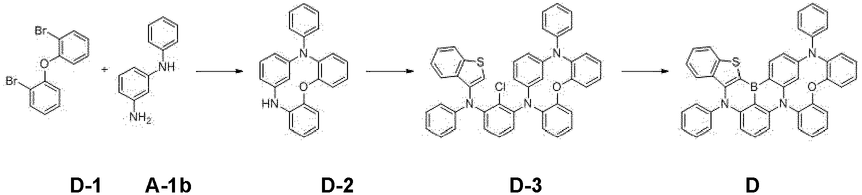

Compound D (yield 20.2%) was synthesized from Intermediate **D-3** in the same manner as in Synthesis Example 1, except that **D-1** (see Angewandte Chemie-International Edition, 2018, vol. 57, # 38, p. 12380-12384 for synthesis) was used instead of Intermediate **A-1a.**
MS (ESI) calcd for Chemical Formula: C₄₄H₂₉BN₃OS (Pos)658.21, found 658.2

### Synthesis Example 5. Preparation of Compound E

### Synthesis Example 5-1. Synthesis of Compound E

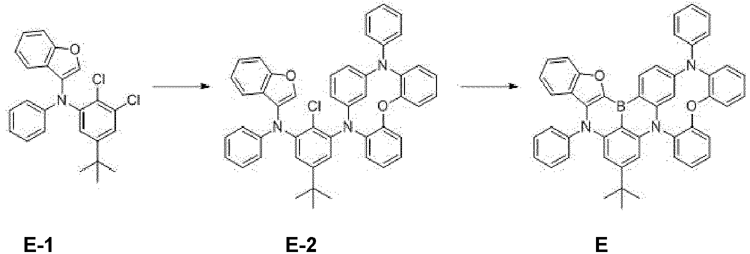

Compound **E** (yield 16.7%) was synthesized from Intermediate E-2 in the same manner as in Synthesis Example 1, except that **E-1** (see US 2020 / 172 558 A1 for synthesis) and **D-2** were used instead of Intermediates **A-2a** and **A-1,** respectively.
MS (ESI) calcd for Chemical Formula: C₄₄H₂₉BN₃O₂ (Pos)642.24, found 642.2

### Synthesis Example 6. Preparation of Compound F

### Synthesis Example 6-1. Synthesis of Intermediate F-1

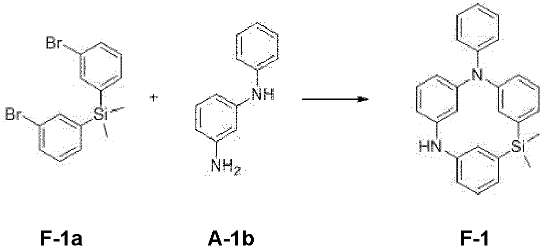

Intermediate **F-1** (yield 30.3%) was synthesized from Intermediate **A-1b** in the same manner as in Synthesis Example 1, except that **F-1a** (see Journal of Organometallic Chemistry, 2013, vol. 735, p. 58-64 for synthesis) was used instead of Intermediate **A-1a.**
MS (ESI) calcd for Chemical Formula: C₂₆H₂₅N₂Si (Pos)393.18, found 393.1

### Synthesis Example 6-2. Synthesis of Compound F

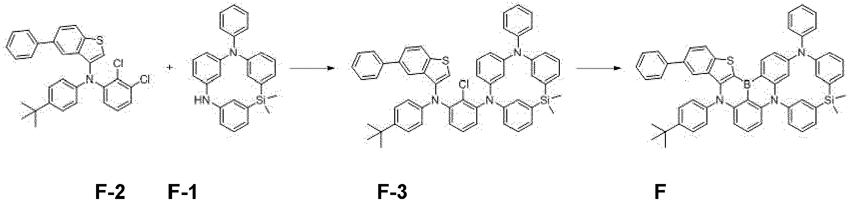

Compound **F** (yield 24.8%) was synthesized from Intermediate **F-3** in the same manner as in Synthesis Example 1, except that **F-2** (see US 2020 / 172 558 A1 for synthesis) and **F-1** were used instead of Intermediates **A-2a** and **A-1,** respectively.
MS (ESI) calcd for Chemical Formula: C₅₆H₄₇BN₃SSi (Pos)832.34, found 832.3

### Synthesis Example 7. Preparation of Compound G

### Synthesis Example 7-1. Synthesis of Intermediate G-1

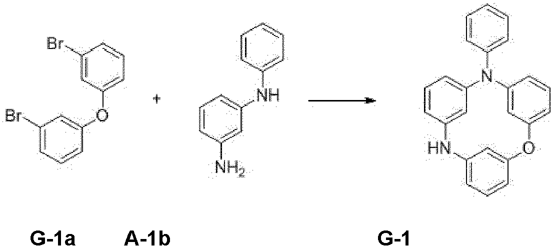

Intermediate **G-1** (yield 33.1%) was synthesized from Intermediate **A-1b** in the same manner as in Synthesis Example 1, except that **G-1a** (see Chemistry-A European Journal, 2010, vol. 16, # 41, p. 12299-12302 for synthesis) was used instead of Intermediate A-1a.
MS (ESI) calcd for Chemical Formula: C₂₄H₁₉N₂O (Pos)351.15, found 351.1

### Synthesis Example 7-2. Synthesis of Compound G

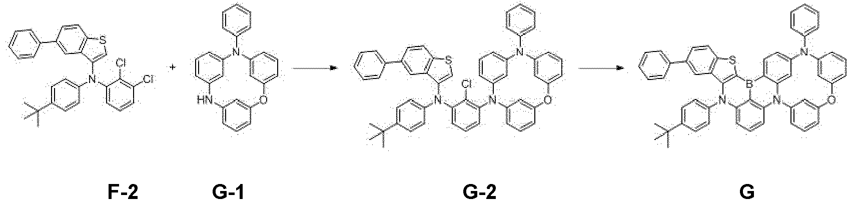

Compound **G** (yield 23.0%) was synthesized from Intermediate **G-2** in the same manner as in Synthesis Example 1, except that **F-2** (see US 2020 / 172 558 A1 for synthesis) and **G-1** were used instead of Intermediates **A-2a** and **A-1,** respectively.
MS (ESI) calcd for Chemical Formula: C₅₄H₄₁BN₃OS (Pos)790.31, found 790.3

### Synthesis Example 8. Preparation of Compound H

### Synthesis Example 8-1. Synthesis of Intermediate H-1

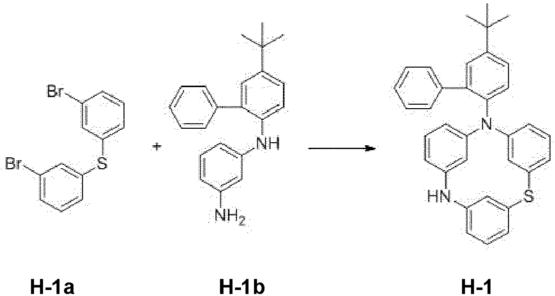

Intermediate **H-1** (yield 17.1%) was synthesized from Intermediate **H-1b** in the same manner as in Synthesis Example 1, except that **H-1a** (see Cell Chemical Biology, 2020, vol. 27, # 8, p. 1063-1072 for synthesis) and **H-1b** were used instead of Intermediates **A-1a** and **A-1b,** respectively.
MS (ESI) calcd for Chemical Formula: C₅₈H₅₁BN₃S (Pos)832.40, found 832.4

### Synthesis Example 8-2. Synthesis of Compound H

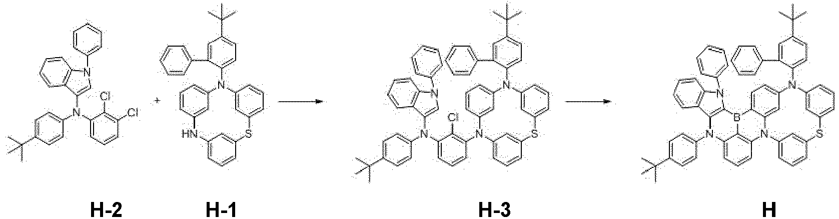

Compound **H** (yield 17.9%) was synthesized from Intermediate **H-3** in the same manner as in Synthesis Example 1, except that **H-2** (see US 2020 / 172 558 A1 for synthesis) and **H-1** were used instead of Intermediates **A-2a** and **A-1,** respectively.
MS (ESI) calcd for Chemical Formula: C₆₄H₅₄BN₄S (Pos)921.42, found 921.4

### Synthesis Example 9. Preparation of Compound I

### Synthesis Example 9-1. Synthesis of Compound I

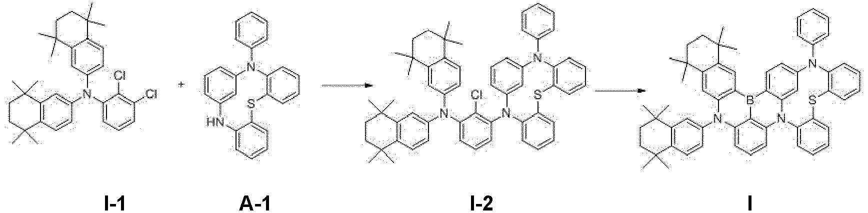

Compound I (yield 22.6%) was synthesized from Intermediate **1-2** in the same manner as in Synthesis Example 1, except that **I-1** (see US 2018 / 0 094 000 A1 for synthesis) was used instead of Intermediate **A-2a.**
MS (ESI) calcd for Chemical Formula: C₅₈H₅₇BN₃S (Pos)838.44, found 838.4

### Synthesis Example 10. Preparation of Compound J

### Synthesis Example 10-1. Synthesis of Intermediate J-1

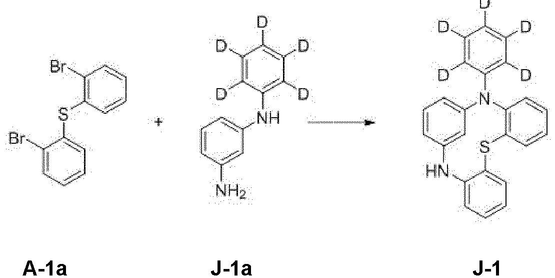

Intermediate **J-1** (yield 30.0%) was synthesized from Intermediate **J-1a** in the same manner as in Synthesis Example 1, except that **J-1a** was used instead of Intermediate **A-1b.**
MS (ESI) calcd for Chemical Formula: C₂₄H₁₄D₅N₂S (Pos)372.16, found 372.1

### Synthesis Example 10-2. Synthesis of Intermediate J-2

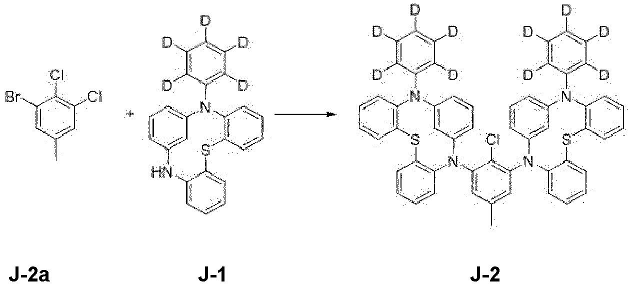

**J-2a** (10.0 g), **J-1** (31.0 g), bis(tri-tert-butylphosphine)palladium(0) (0.43 g), sodium tert-butoxide (8.0 g), and toluene (100 mL) were placed in a reactor. The mixture was stirred under reflux for 18 h. The reaction mixture was cooled to room temperature and water (40 mL) was added thereto. The organic layer was extracted with ethyl acetate, concentrated under reduced pressure, and purified by silica gel column chromatography to afford **J-2** (29.9 g, yield 82.9%).
MS (ESI) calcd for Chemical Formula: C₅₅H₃₀D₁₀ClN₄S₂ (Pos)865.30, found 865.3

### Synthesis Example 10-3. Synthesis of Compound J

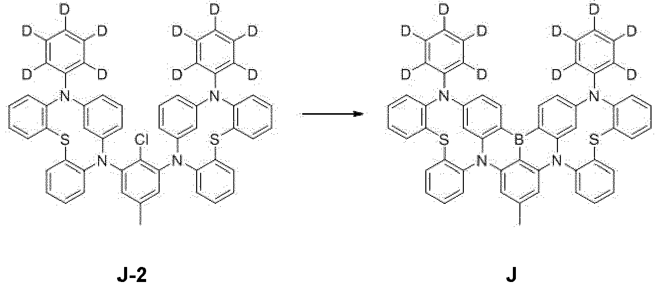

Compound **J** (yield 24.8%) was synthesized from Intermediate **J-2** in the same manner as in Synthesis Example 1.
MS (ESI) calcd for Chemical Formula: C₅₅H₂₈D₁₀BN₄S₂ (Pos)839.33, found 839.3

### Synthesis Example 11. Preparation of Compound K

### Synthesis Example 11-1. Synthesis of Intermediate K-1

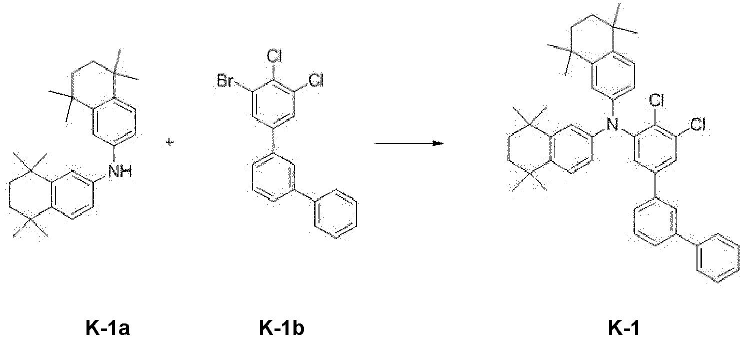

**K-1a** (15.0 g) (see Tetrahedron, 2014, vol. 70, # 32, p. 4754-4759 for synthesis), **K-1b** (29.1 g) (see CN 106 674 210 and US 2020 / 172 558 A1 for synthesis), bis(tri-tert-butylphosphine)palladium(0) (0.39 g), sodium tert-butoxide (7.4 g), and toluene (150 mL) were placed in a reactor. The mixture was stirred under reflux for 12 h. The reaction mixture was cooled to room temperature and water (60 mL) was added thereto. The organic layer was extracted with ethyl acetate, concentrated under reduced pressure, and purified by silica gel column chromatography to afford **K-1** (19.3 g, yield 73.0%).
MS (ESI) calcd for Chemical Formula: C₄₆H₅₀Cl₂N (Pos)686.33, found 686.3

### Synthesis Example 11-2. Synthesis of Intermediate K-2

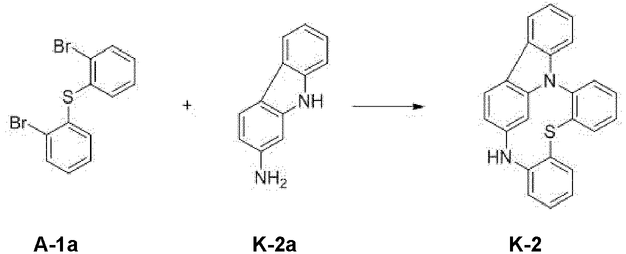

Intermediate **K-2** (yield 21.4%) was synthesized from Intermediate **K-2a** in the same manner as in Synthesis Example 1, except that **K-2a** (see Tetrahedron Letters, 2018, vol. 59, # 22, p. 2145-2149 for synthesis) was used instead of Intermediate **A-2a.**
MS (ESI) calcd for Chemical Formula: C₂₄H₁₇N₂S (Pos)365.11, found 365.1

### Synthesis Example 11-3. Synthesis of Intermediate K-3

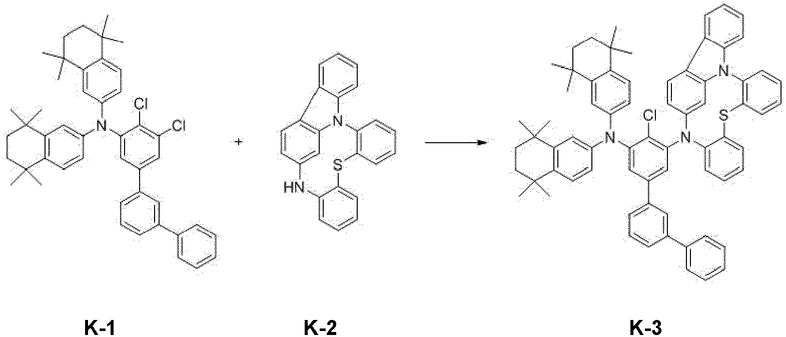

Intermediate **K-3** (yield 79.5%) was synthesized from Intermediate **K-1** in the same manner as in Synthesis Example 1.
MS (ESI) calcd for Chemical Formula: C₇₀H₆₅ClN₃S (Pos)1014.46, found 1014.4

### Synthesis Example 11-4. Synthesis of Compound K

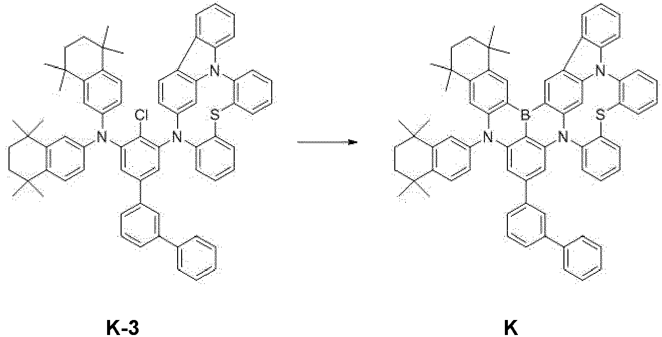

Compound K (yield 19.5%) was synthesized from Intermediate **K-3** in the same manner as in Synthesis Example 1.
MS (ESI) calcd for Chemical Formula: C₇₀H₆₃BN₃S (Pos)988.49, found 988.4

### Synthesis Example 12. Preparation of Compound L

### Synthesis Example 12-1. Synthesis of Intermediate L-1

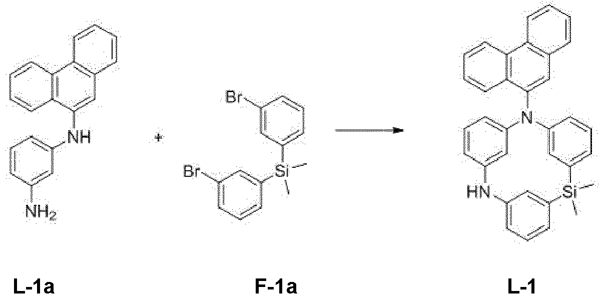

Intermediate **L-1** (yield 28.9%) was synthesized from Intermediates **L-1a** and **F-1a** (see Tetrahedron, 2019, vol. 75, # 6, p. 721-731 for synthesis) in the same manner as in Synthesis Example 1.
MS (ESI) calcd for Chemical Formula: C₃₄H₂₉N₂Si (Pos)493.21, found 493.2

### Synthesis Example 12-2. Synthesis of Intermediate L-2

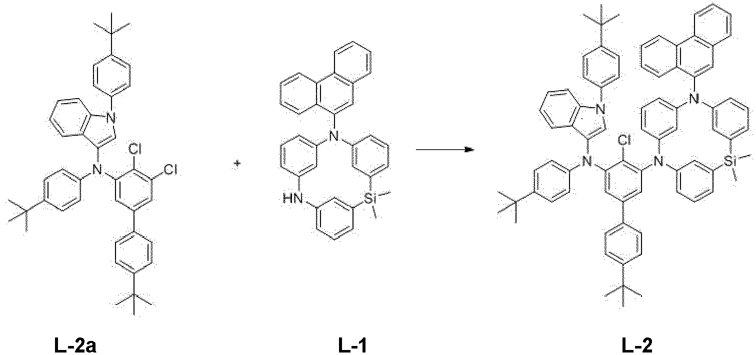

Intermediate **L-2** (yield 81.8%) was synthesized from Intermediates **L-2a** (see US 2020 / 172 558 A1 for synthesis) and **L-1** in the same manner as in Synthesis Example 1.
MS (ESI) calcd for Chemical Formula: C₇₈H₇₄Cl₄Si (Pos)1129.54, found 1129.5

### Synthesis Example 12-3. Synthesis of Compound L

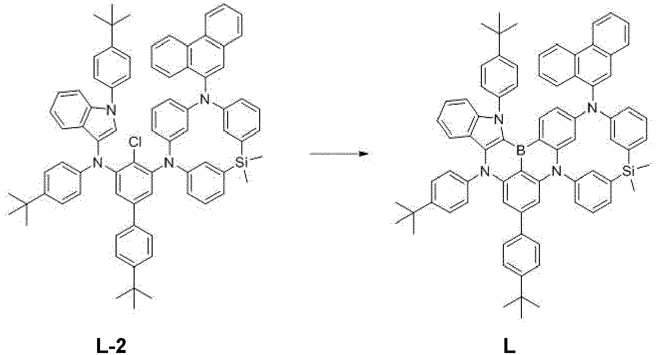

Compound **L** (yield 23.8%) was synthesized from Intermediate **L-2** in the same manner as in Synthesis Example 1.
MS (ESI) calcd for Chemical Formula: C₇₈H₇₁BN₄Si (Pos)1103.56, found 1103.5

### Synthesis Example 13. Preparation of Compound M

### Synthesis Example 13-1. Synthesis of Intermediate M-1

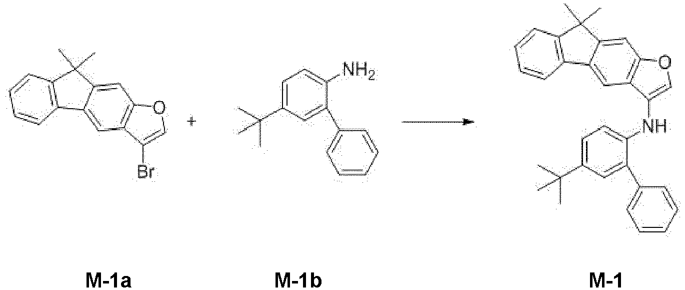

**M-1a** (23.0 g) (see Chinese Patent Publication No. 111303149A for synthesis), **M-1b** (16.6 g) (see Angewandte Chemie-International Edition, 2015, vol. 54, # 51, p. 15385-15389 for synthesis), tris(dibenzylideneacetone)dipalladium(0) (1.34 g), bis(diphenylphosphino)-1,1'-binaphthyl (0.91 g), sodium tert-butoxide (14.1 g), and toluene (230 mL) were placed in a reactor. The mixture was stirred under reflux for 18 h. The reaction mixture was cooled to room temperature and water (100 mL) was added thereto. Thereafter, the organic layer was separated and purified by silica gel column chromatography to afford **M-1** (28.1 g, yield 83.6%).
MS (ESI) calcd for Chemical Formula: C₃₃H₃₂NO (Pos)458.25, found 458.2

### Synthesis Example 13-2. Synthesis of Intermediate M-2

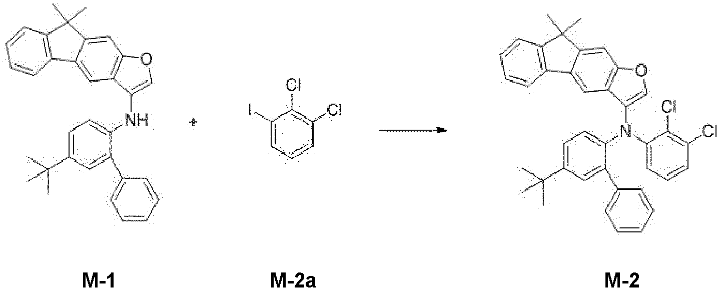

Compound **M-2** (yield 42.7%) was synthesized from Intermediates **M-1** and **M-2a** in the same manner as in Synthesis Example 11.
MS (ESI) calcd for Chemical Formula: C₃₉H₃₄Cl₂NO (Pos)602.20, found 602.2

### Synthesis Example 13-3. Synthesis of intermediate M-3

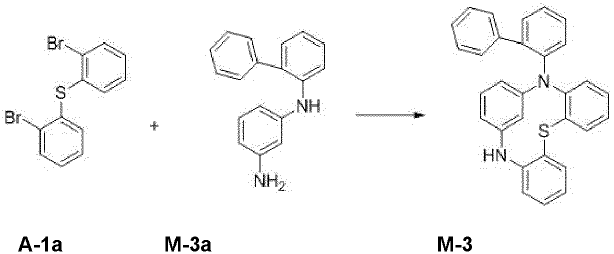

Intermediate **M-3** (yield 25.7%) was synthesized from Intermediate **M-3a** in the same manner as in Synthesis Example 1, except that **M-3a** (see Tetrahedron, 2019, vol. 75, # 6, p. 721-731 for synthesis) was used instead of Intermediate **A-1b.**
MS (ESI) calcd for Chemical Formula: C₃₀H₂₃N₂S (Pos)443.16, found 443.1

### Synthesis Example 13-4. Synthesis of Intermediate M-4

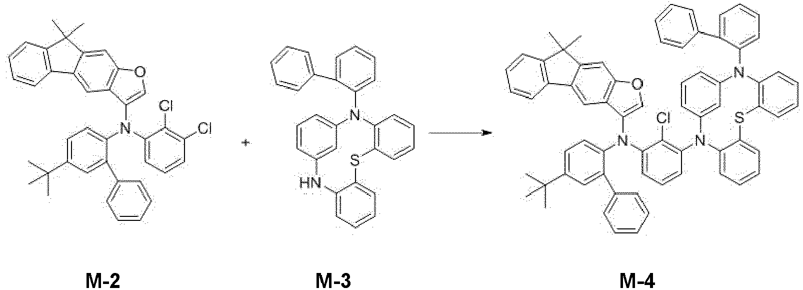

Intermediate **M-4** (yield 80.0%) was synthesized from Intermediates **M-2** and **M-3** in the same manner as in Synthesis Example 1.
MS (ESI) calcd for Chemical Formula: C₆₉H₅₅ClN₃OS (Pos)1008.38, found 1008.3

### Synthesis Example 13-5. Synthesis of Compound M

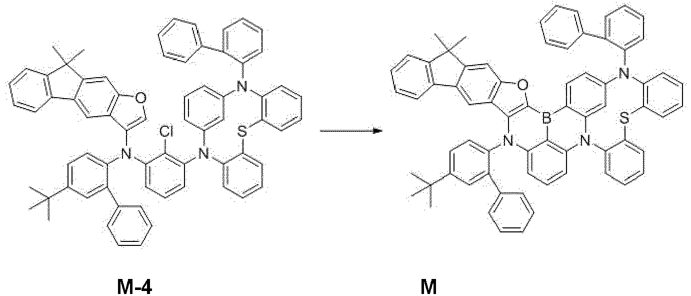

Compound **M** (yield 22.3%) was synthesized from Intermediate **M-4** in the same manner as in Synthesis Example 1.
MS (ESI) calcd for Chemical Formula: C₆₉H₅₃BN₃OS (Pos)982.40, found 982.4

### Examples 1-10: Fabrication of organic electroluminescent devices

ITO glass was patterned to have a light emitting area of 2 mm × 2 mm, followed by cleaning. After the cleaned ITO glass was mounted in a vacuum chamber, the base pressure was adjusted to 1 × 10⁻⁷ torr. 4,4',4"-tris[2-naphthyl(phenyl)amino]triphenylamine (2-TNATA) (700 Å) and the compound represented by Formula G (250 Å) were deposited in this order on the ITO. A mixture of the compound represented by BH1 as a host and the inventive compound shown in Table 1 in a weight ratio of 98:2 was used to form a 250 Å thick light emitting layer. Thereafter, a mixture of the compound represented by Formula E-1 and the compound represented by Formula E-2 in a ratio of 1:1 was used to form a 300 Å thick electron transport layer on the light emitting layer. The compound represented by Formula E-1 was used to form a 5 Å thick electron injecting layer on the electron transport layer. Al was used to form a 1000 Å thick Al electrode on the electron injecting layer, completing the fabrication of an organic electroluminescent device. The luminescent properties of the organic electroluminescent device were measured at 0.4 mA.

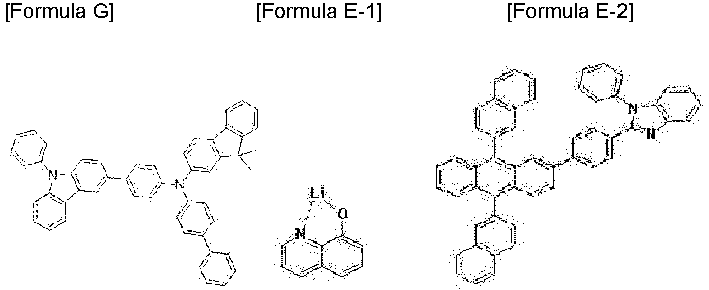

### Comparative Examples 1-2

An organic electroluminescent device (Comparative Example 1) was fabricated in the same manner as in Examples 1-10, except that BD1 was used as a dopant compound instead of the inventive compound. An organic electroluminescent device (Comparative Example 2) was fabricated in the same manner as in Examples 1-10, except that BH2 was used as a host compound and BD1 was used as a dopant compound instead of the inventive compound. The luminescent properties of the organic electroluminescent devices were measured at 0.4 mA. The structures of BH1, BH2, and BD1 are as follow:

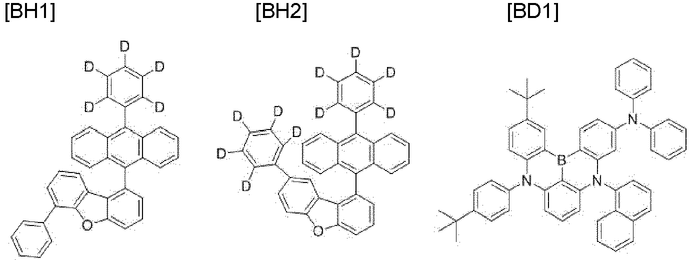

The organic electroluminescent devices of Examples 1-10 and Comparative Examples 1-2 were measured for voltage, external quantum efficiency, and lifetime. The results are shown in Table 1.

**[Table 1]**

| Example No. | Host | Dopant | Voltage (V) | External quantum efficiency (%) | Lifetime (LT97) |
|---|---|---|---|---|---|
| Example 1 | BH1 | Compound F | 3.7 | 9.21 | 149 |
| Example 2 | BH1 | Compound G | 3.7 | 9.14 | 181 |
| Example 3 | BH1 | Compound H | 3.7 | 9.01 | 173 |
| Example 4 | BH1 | Compound K | 3.7 | 9.31 | 169 |
| Example 5 | BH1 | Compound L | 3.7 | 9.01 | 143 |
| Example 6 | BH1 | Compound J | 3.7 | 8.98 | 158 |
| Example 7 | BH1 | Compound M | 3.7 | 9.05 | 151 |
| Example 8 | BH1 | Compound O | 3.7 | 9.07 | 160 |
| Example 9 | BH1 | Compound P | 3.7 | 9.11 | 157 |
| Example 10 | BH1 | Compound N | 3.7 | 9.03 | 166 |
| Comparative Example 1 | BH1 | BD1 | 3.7 | 8.56 | 105 |
| Comparative Example 2 | BH2 | BD1 | 3.5 | 8.07 | 98 |

As can be seen from the results in Table 1, the organic electroluminescent devices of Examples 1-10, each of which employed the compound of Formula I or II as a dopant compound to form the light emitting layer, showed significantly improved efficiencies and life characteristics compared to the devices of Comparative Examples 1-2 employing BD1 whose structural features were contrasted with those of the inventive compound. These results concluded that the use of the inventive compounds makes the organic electroluminescent devices highly efficient and long lasting.

### Industrial Applicability

The polycyclic aromatic derivative of the present invention can be employed in an organic layer of an organic electroluminescent device to achieve high efficiency and long lifetime of the device. Therefore, the polycyclic aromatic derivative of the present invention can find useful industrial applications in various displays, including flat panel displays and flexible displays, and lighting systems, including monochromatic flat panel lighting systems, white flat panel lighting systems, flexible monochromatic lighting systems, and flexible white lighting systems.

## Claims

1. An organic compound represented by Formula I or II: 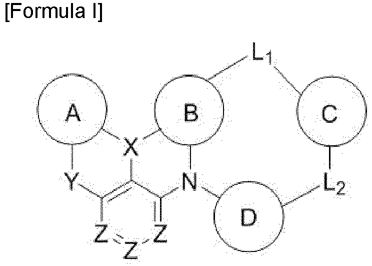 wherein the rings A to D are the same as or different from each other and are each independently a substituted or unsubstituted C₆-C₅₀ monocyclic or polycyclic aromatic hydrocarbon ring or a substituted or unsubstituted C₂-C₅₀ monocyclic or polycyclic aromatic heterocyclic ring, X is selected from B, P, P=O, P=S, and Al, L₁ and L₂ are the same as or different from each other and are each independently a single bond or selected from 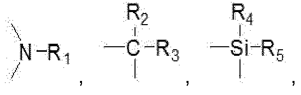 -O-, -S-, and -Se-, with the proviso that L₂ linking the rings C and D is provided in one or two and the two linkers L₂ are the same as or different from each other and optionally form a ring with the rings C and D, Y is a single bond or selected from 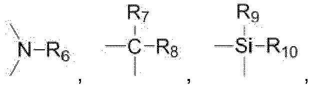 -O-, -S-, and -Se-, each Z is independently CR or N, R and R₁ to R₁₀ are the same as or different from each other and are each independently selected from hydrogen, deuterium, substituted or unsubstituted C₁-C₃₀ alkyl, substituted or unsubstituted C₁-C₃₀ alkenyl, substituted or unsubstituted C₆-C₅₀ aryl, substituted or unsubstituted C₃-C₃₀ cycloalkyl, substituted or unsubstituted C₂-C₃₀ heterocycloalkyl, substituted or unsubstituted C₂-C₅₀ heteroaryl, substituted or unsubstituted C₁-C₃₀ alkoxy, substituted or unsubstituted C₆-C₃₀ aryloxy, substituted or unsubstituted C₁-C₃₀ alkylthioxy, substituted or unsubstituted C₅-C₃₀ arylthioxy, substituted or unsubstituted amine, substituted or unsubstituted silyl, nitro, cyano, and halogen, with the proviso that R₁ is optionally bonded to the adjacent ring C or D to form an alicyclic or aromatic monocyclic or polycyclic ring, each of R₆ to R₁₀ is optionally bonded to the ring A or R to form an alicyclic or aromatic monocyclic or polycyclic ring, R is optionally bonded to the ring D to form an alicyclic or aromatic monocyclic or polycyclic ring, R₂ and R₃ are optionally linked to each other to form an alicyclic or aromatic monocyclic or polycyclic ring, R₄ and R₅ are optionally linked to each other to form an alicyclic or aromatic monocyclic or polycyclic ring, R₇ and R₈ are optionally linked to each other to form an alicyclic or aromatic monocyclic or polycyclic ring, R₉ and R₁₀ are optionally linked to each other to form an alicyclic or aromatic monocyclic or polycyclic ring, a substituted or unsubstituted monocyclic or polycyclic non-aromatic ring is optionally fused to the ring A to form a fused ring, a substituted or unsubstituted monocyclic or polycyclic non-aromatic ring is optionally fused to the ring B to form a fused ring, a substituted or unsubstituted monocyclic or polycyclic non-aromatic ring is optionally fused to the ring C to form a fused ring, a substituted or unsubstituted monocyclic or polycyclic non-aromatic ring is optionally fused to the ring D to form a fused ring, and the rings A and B are optionally linked to each other, 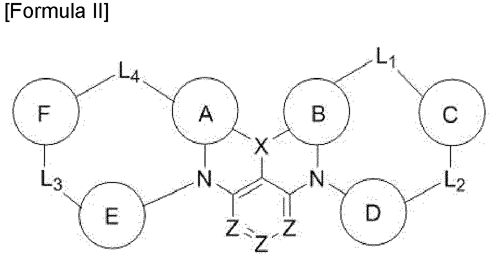 wherein the rings A to F are the same as or different from each other and are each independently a substituted or unsubstituted C₆-C₅₀ monocyclic or polycyclic aromatic hydrocarbon ring or a substituted or unsubstituted C₂-C₅₀ monocyclic or polycyclic aromatic heterocycle, X is selected from B, P, P=O, P=S, and Al, L₁ to L₄ are the same as or different from each other and are each independently a single bond or selected from 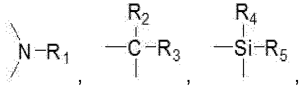 -O-, -S-, and -Se-, with the proviso that L₂ linking the rings C and D is provided in one or two and the two linkers L₂ are the same as or different from each other and optionally form a ring with the rings C and D and that L₃ linking the rings E and F is provided in one or two and the two linkers L₃ are the same as or different from each other and optionally form a ring with the rings E and F, each Z is independently CR or N, R and R₁ to R₅ are the same as or different from each other and are each independently selected from hydrogen, deuterium, substituted or unsubstituted C₁-C₃₀ alkyl, substituted or unsubstituted C₁-C₃₀ alkenyl, substituted or unsubstituted C₆-C₅₀ aryl, substituted or unsubstituted C₃-C₃₀ cycloalkyl, substituted or unsubstituted C₂-C₃₀ heterocycloalkyl, substituted or unsubstituted C₂-C₅₀ heteroaryl, substituted or unsubstituted C₁-C₃₀ alkoxy, substituted or unsubstituted C₆-C₃₀ aryloxy, substituted or unsubstituted C₁-C₃₀ alkylthioxy, substituted or unsubstituted C₅-C₃₀ arylthioxy, substituted or unsubstituted amine, substituted or unsubstituted silyl, nitro, cyano, and halogen, with the proviso that R₁ is optionally bonded to the adjacent ring C, D, E or F to form an alicyclic or aromatic monocyclic or polycyclic ring, R is optionally bonded to the ring D or E to form an alicyclic or aromatic monocyclic or polycyclic ring, R₂ and R₃ are optionally linked to each other to form an alicyclic or aromatic monocyclic or polycyclic ring, R₄ and R₅ are optionally linked to each other to form an alicyclic or aromatic monocyclic or polycyclic ring, a substituted or unsubstituted monocyclic or polycyclic non-aromatic ring is optionally fused to the ring A to form a fused ring, a substituted or unsubstituted monocyclic or polycyclic non-aromatic ring is optionally fused to the ring B to form a fused ring, a substituted or unsubstituted monocyclic or polycyclic non-aromatic ring is optionally fused to the ring C to form a fused ring, a substituted or unsubstituted monocyclic or polycyclic non-aromatic ring is optionally fused to the ring D to form a fused ring, a substituted or unsubstituted monocyclic or polycyclic non-aromatic ring is optionally fused to the ring E to form a fused ring, a substituted or unsubstituted monocyclic or polycyclic non-aromatic ring is optionally fused to the ring F to form a fused ring, and the rings A and B are optionally linked to each other.

2. The organic compound according to claim 1, wherein the organic compound is represented by Formula I-1, II-1, II-2 or II-3:

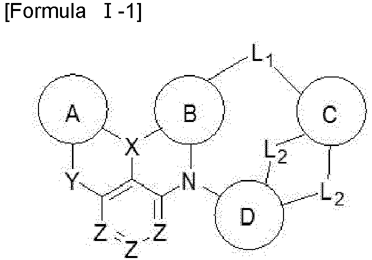

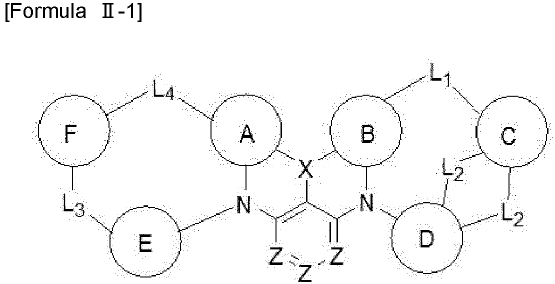

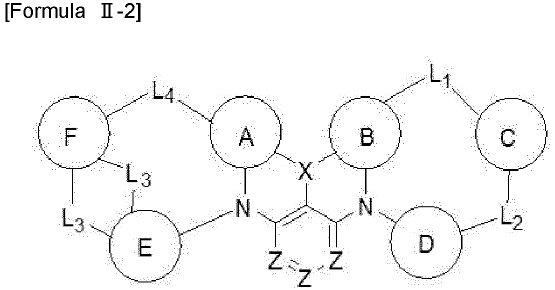

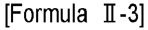

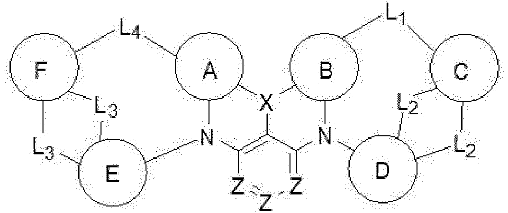

3. The organic compound according to claim 1, wherein the organic compound is selected from the following compounds:

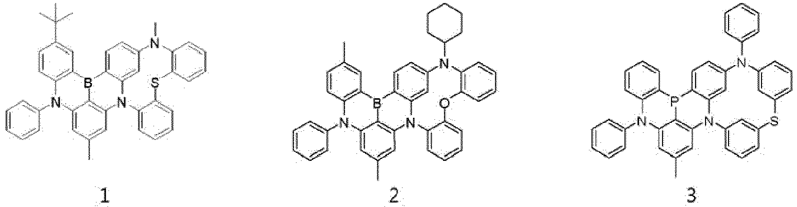

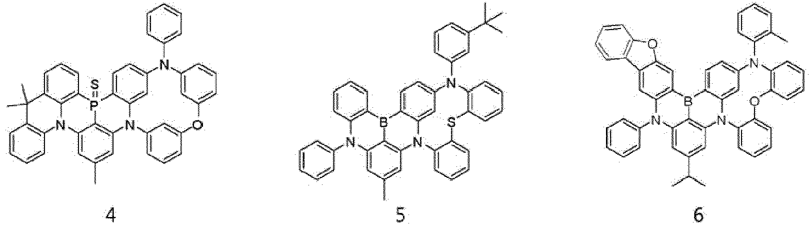

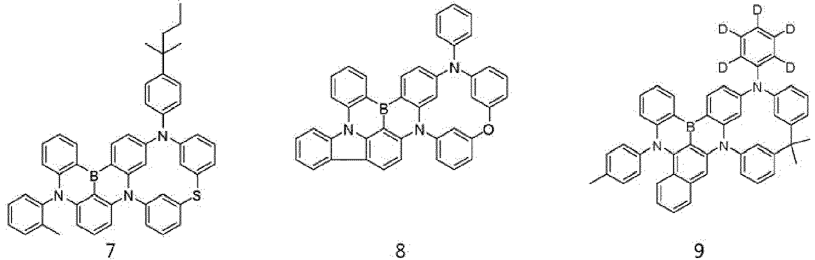

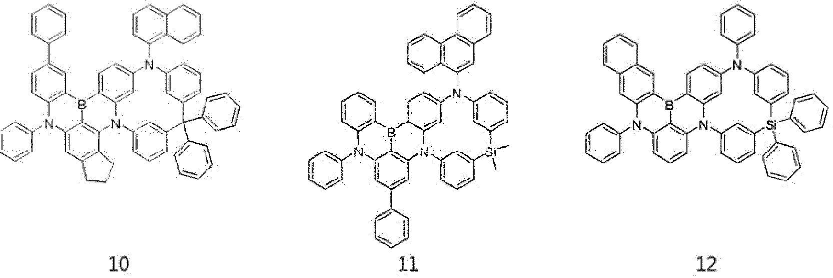

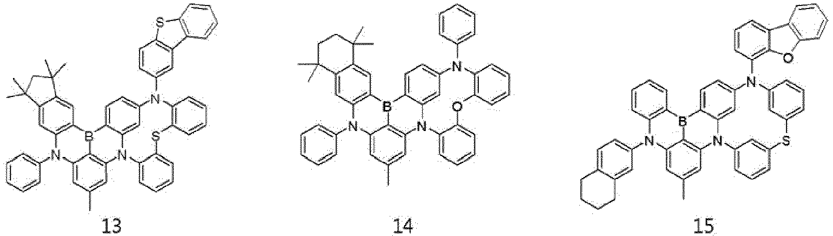

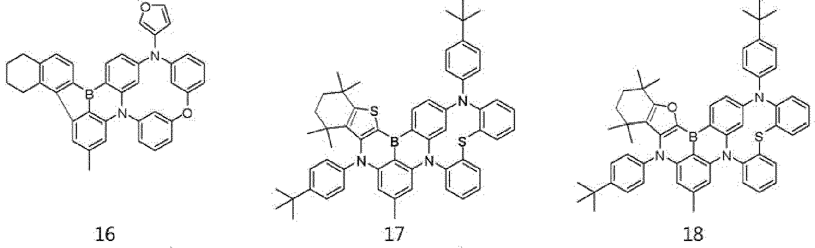

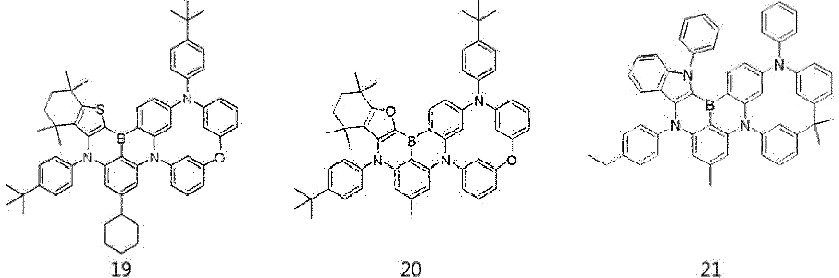

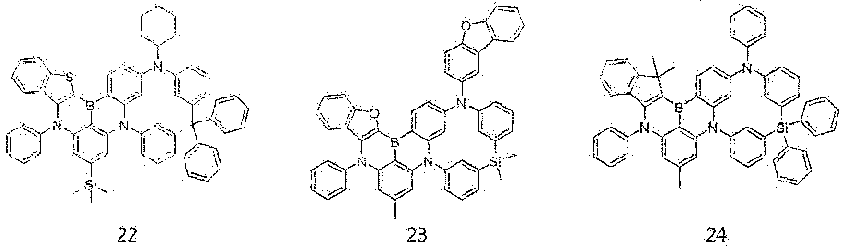

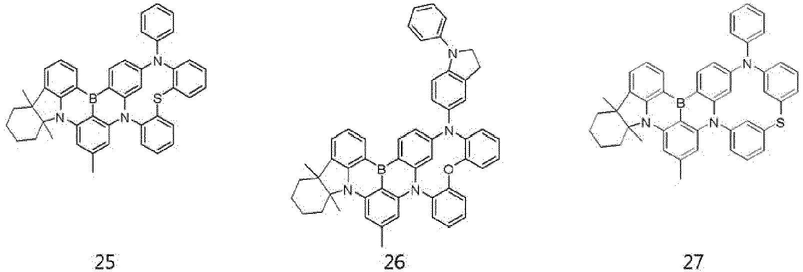

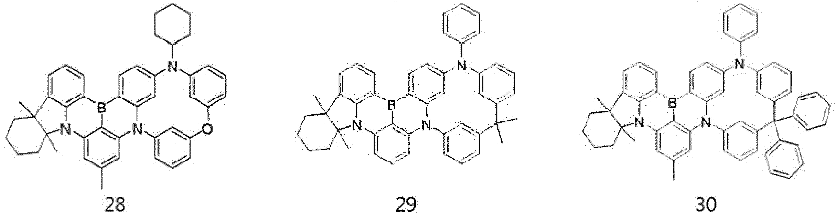

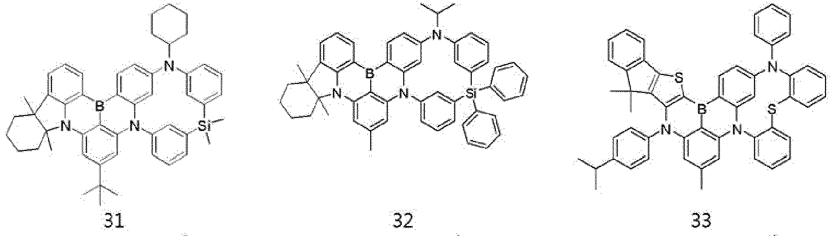

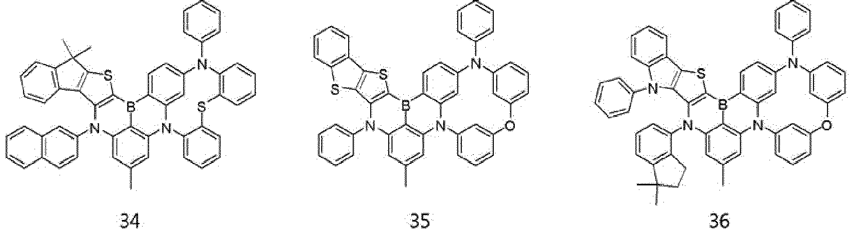

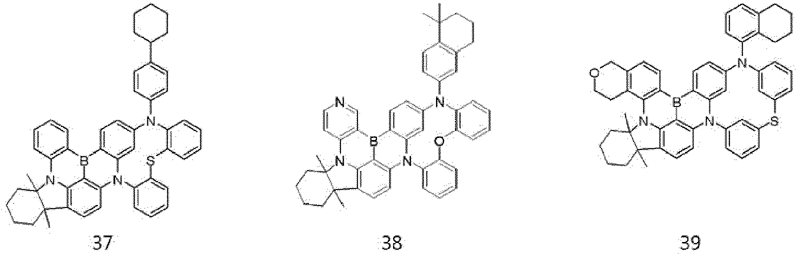

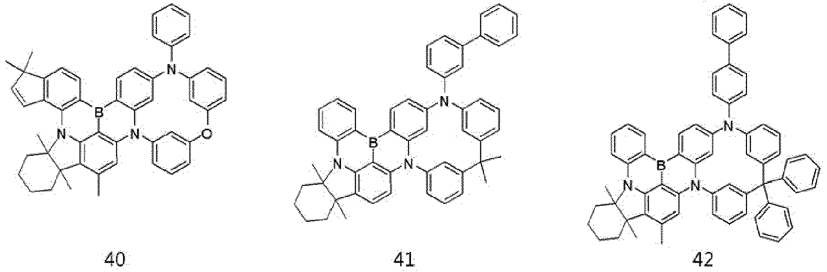

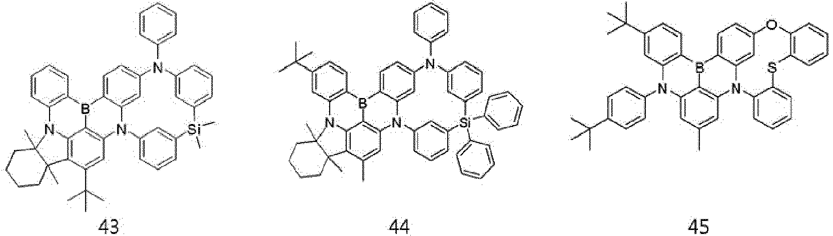

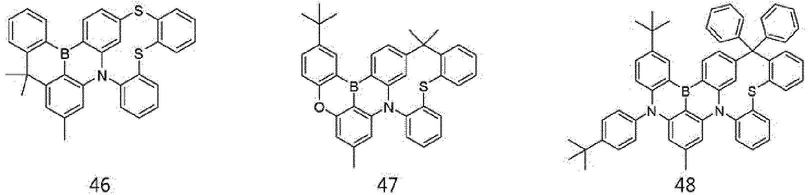

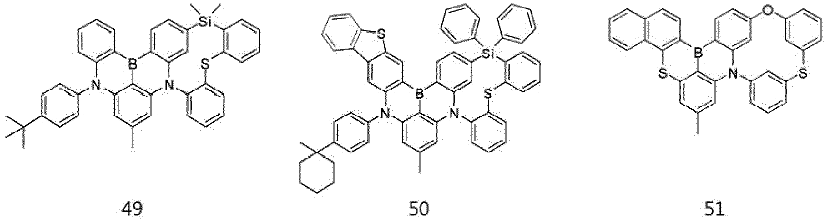

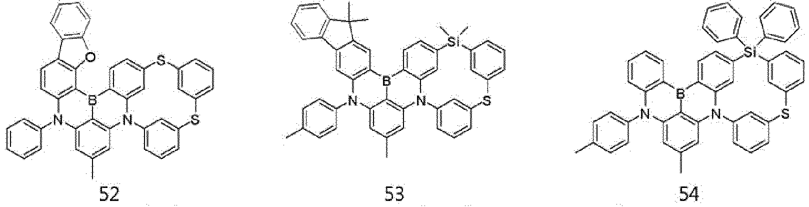

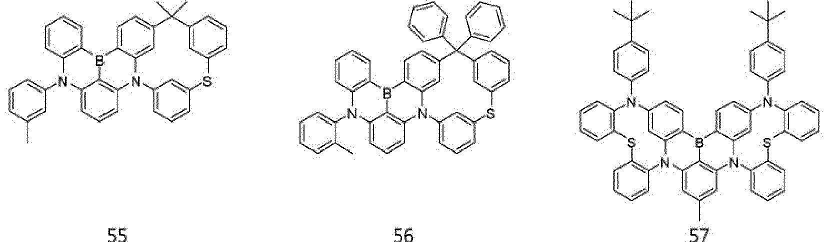

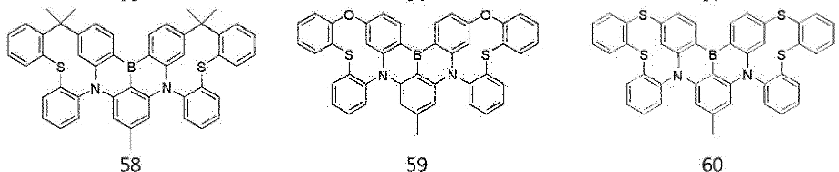

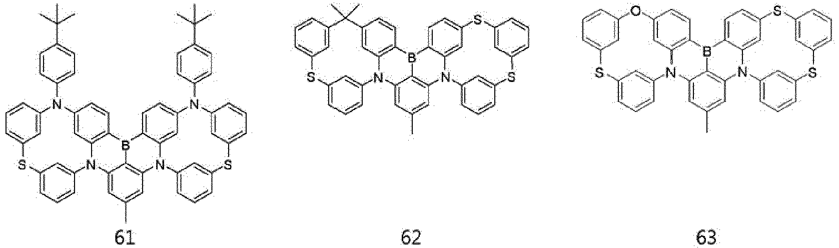

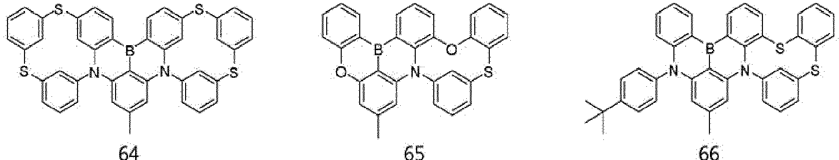

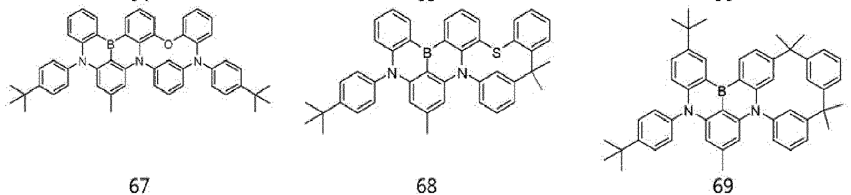

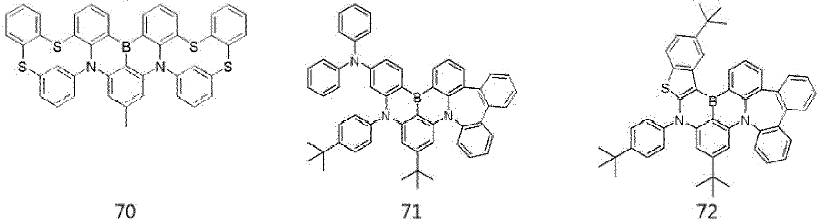

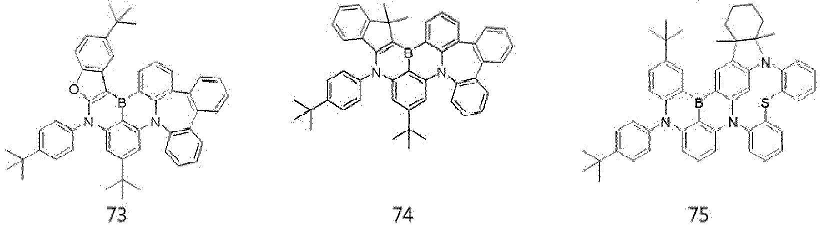

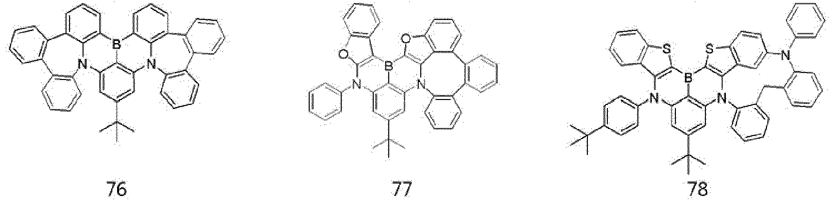

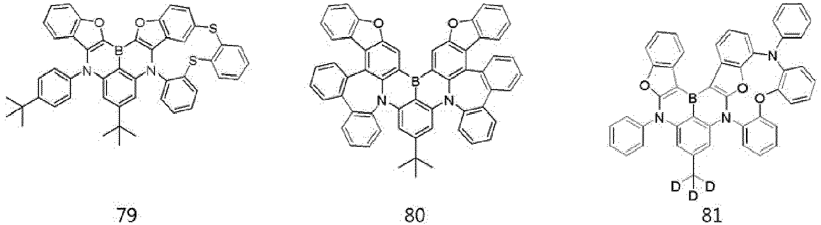

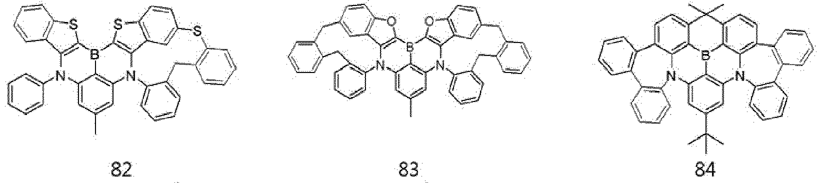

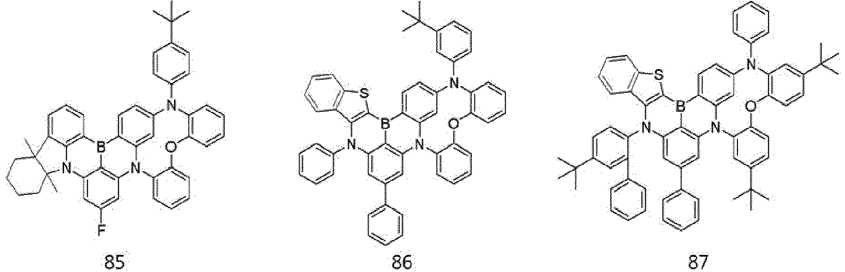

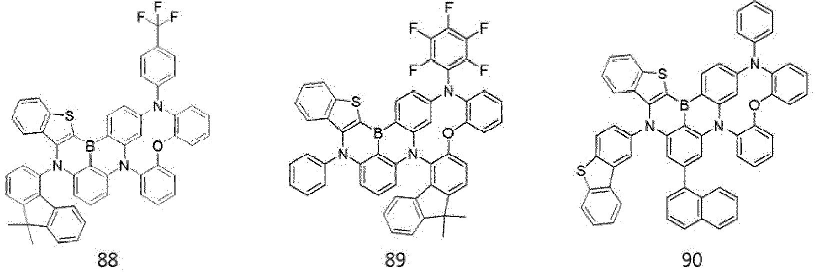

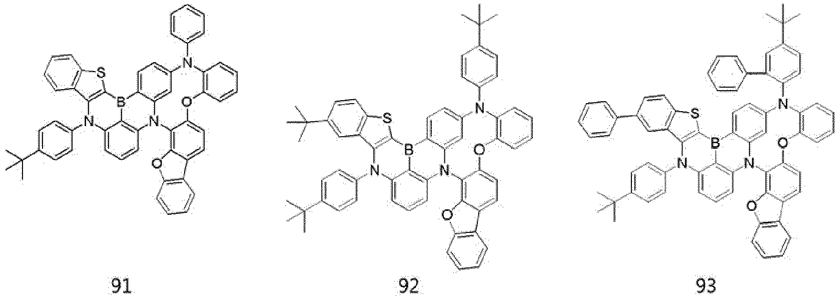

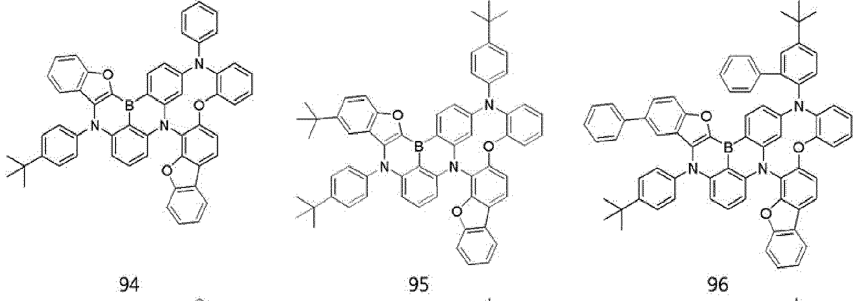

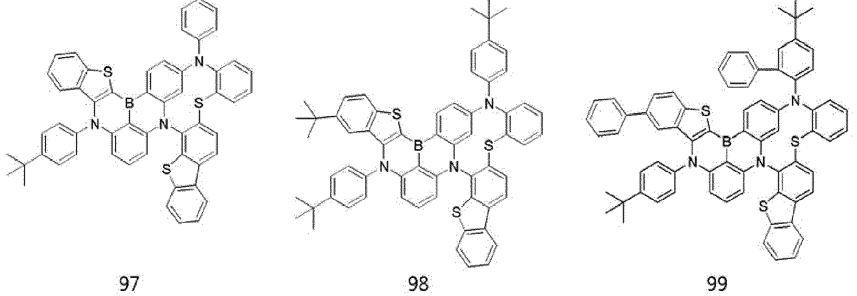

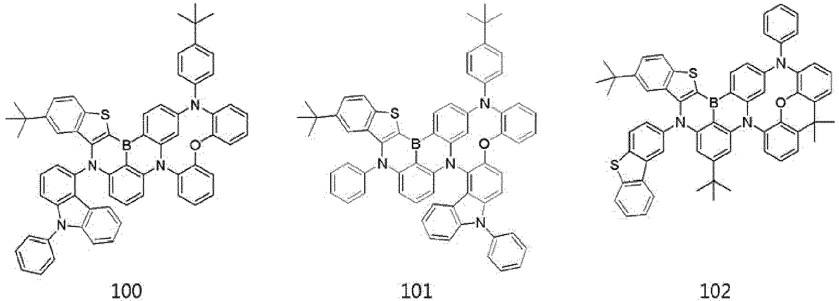

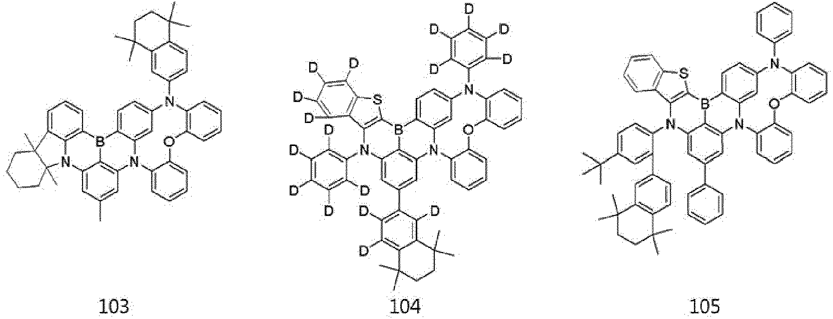

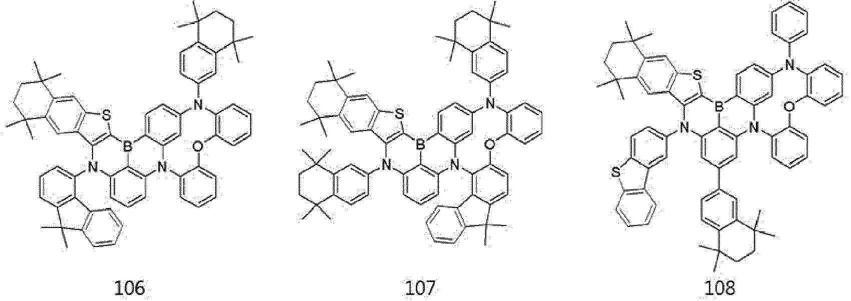

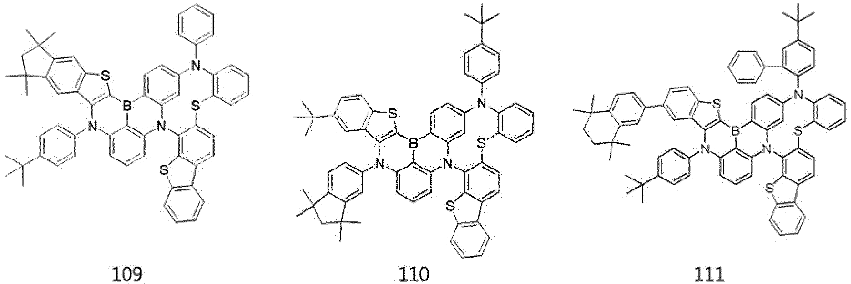

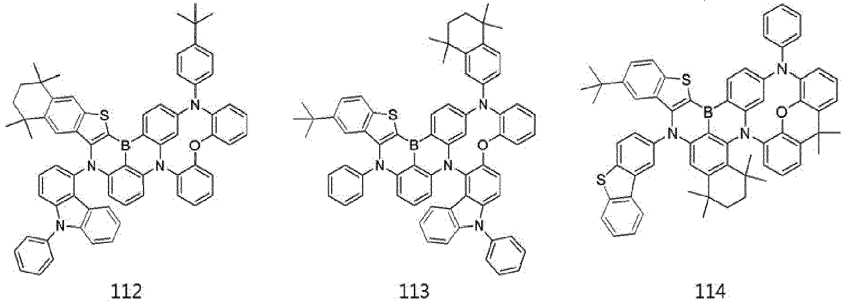

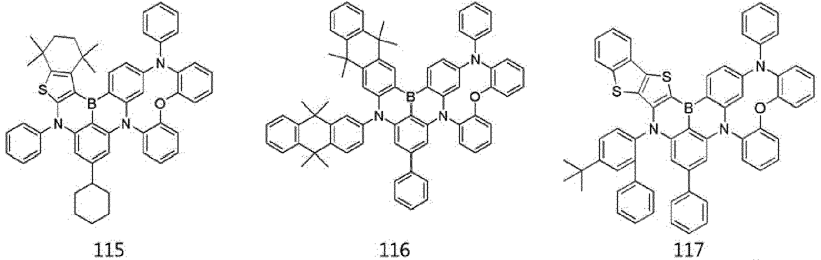

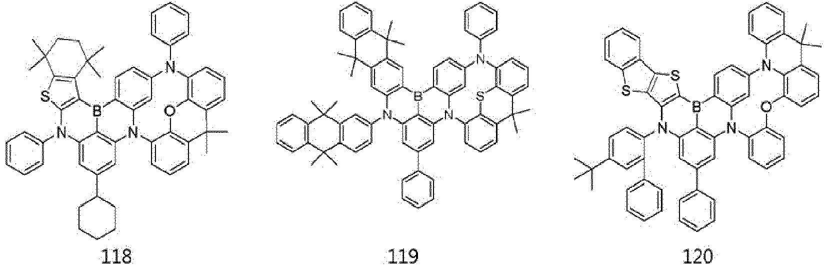

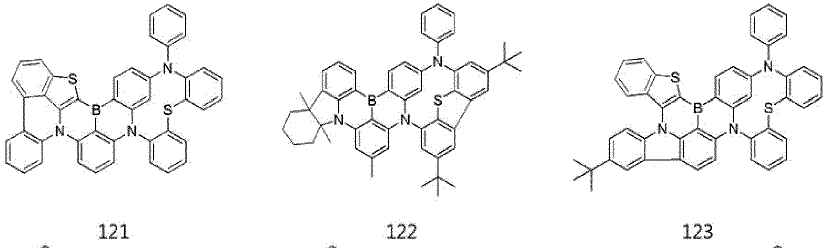

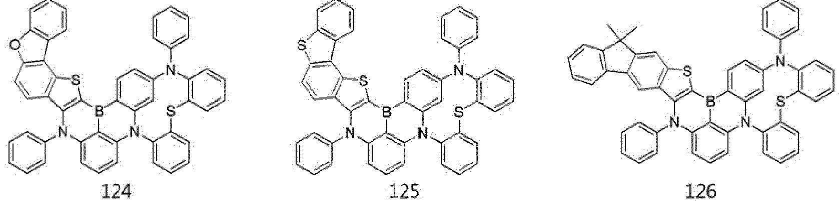

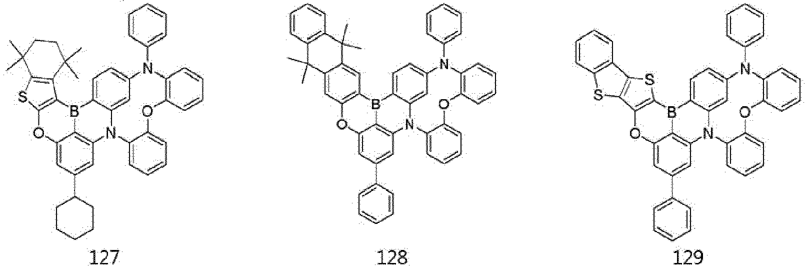

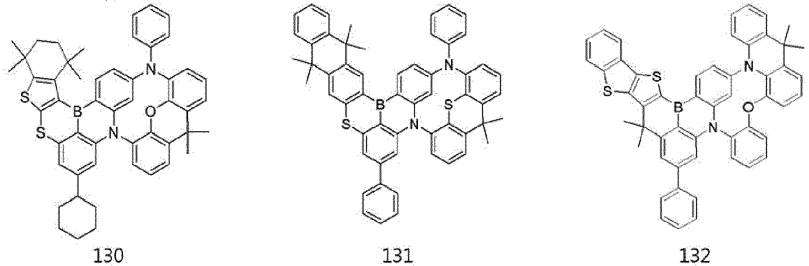

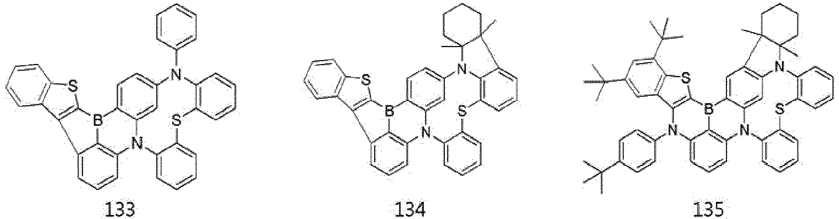

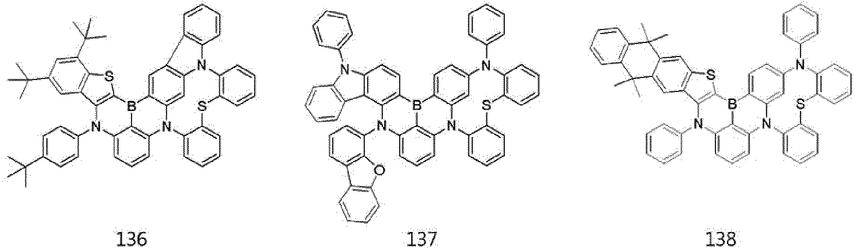

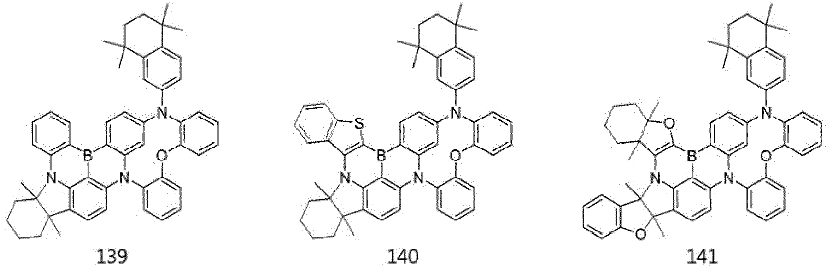

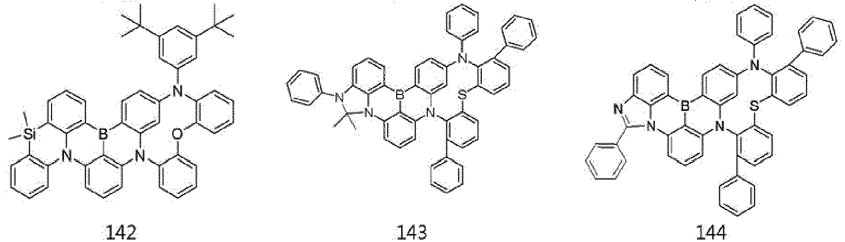

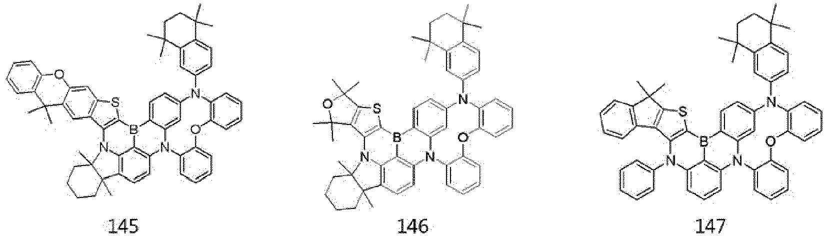

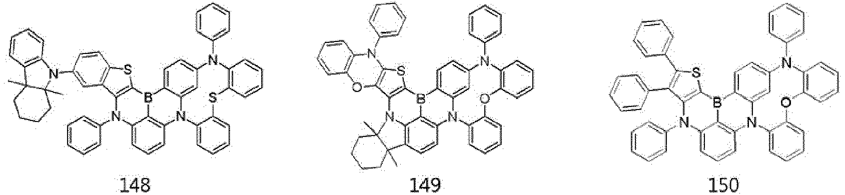

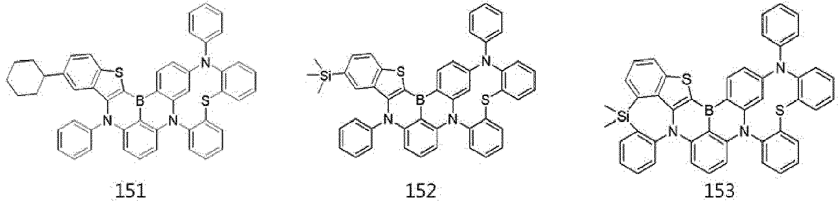

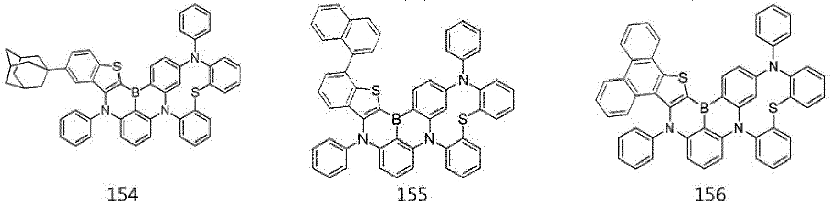

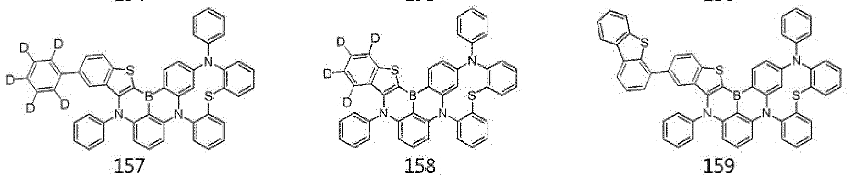

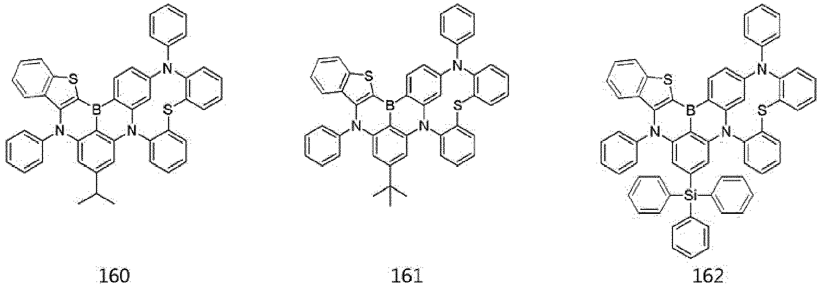

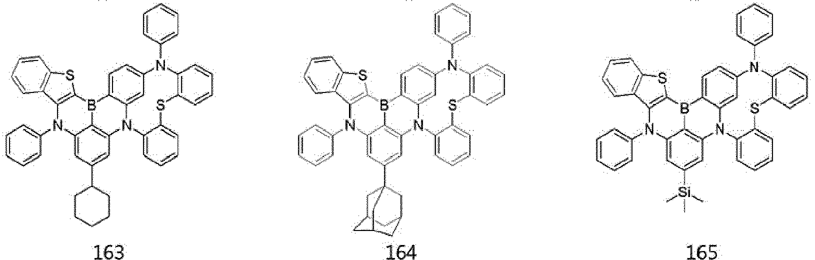

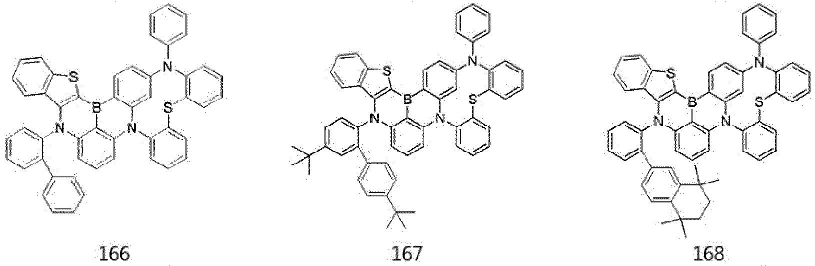

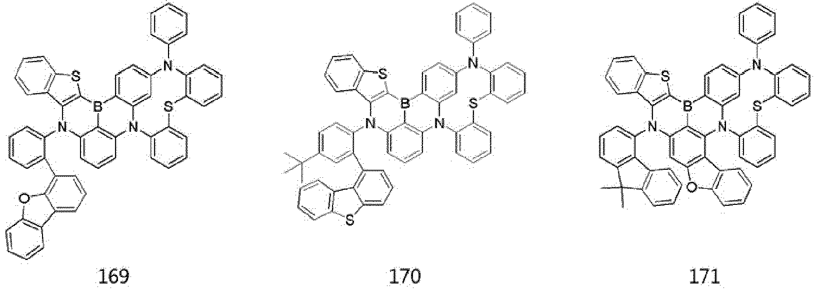

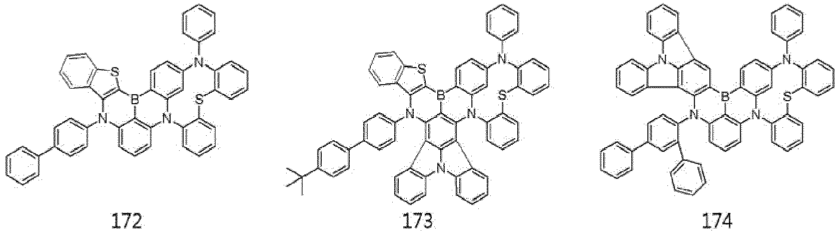

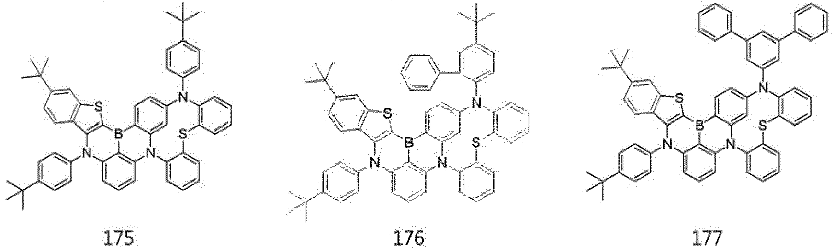

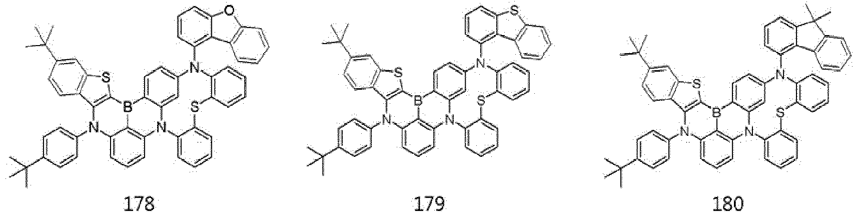

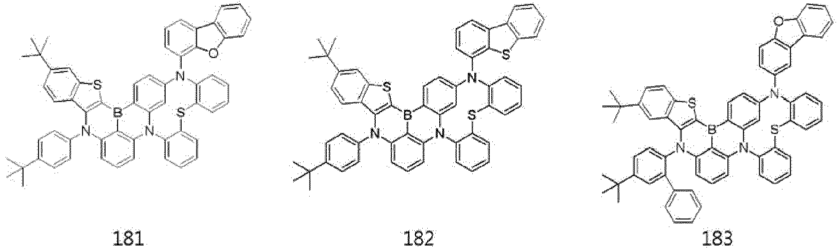

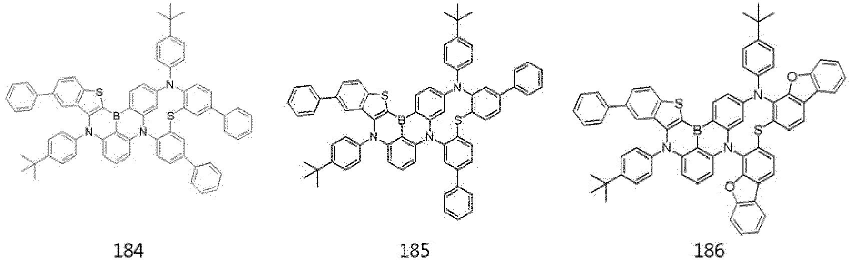

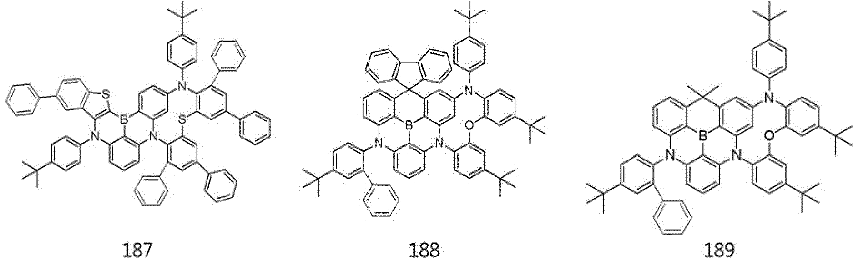

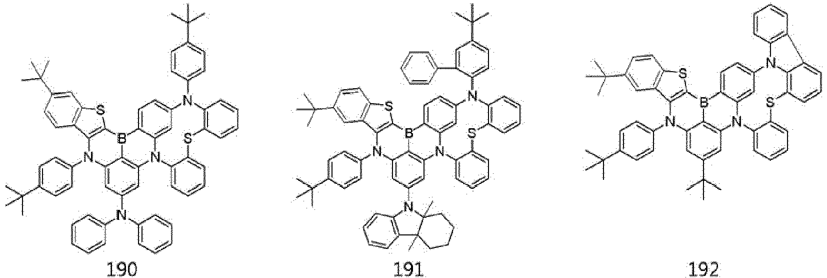

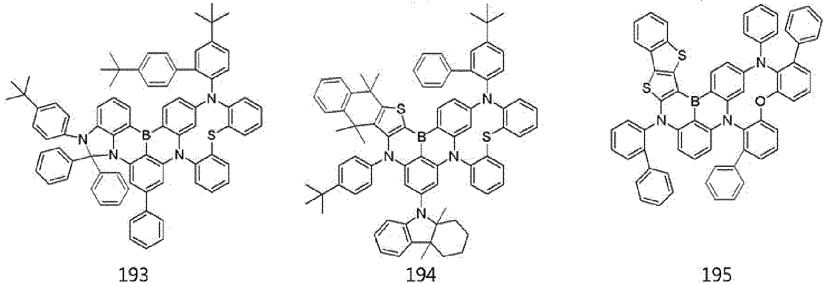

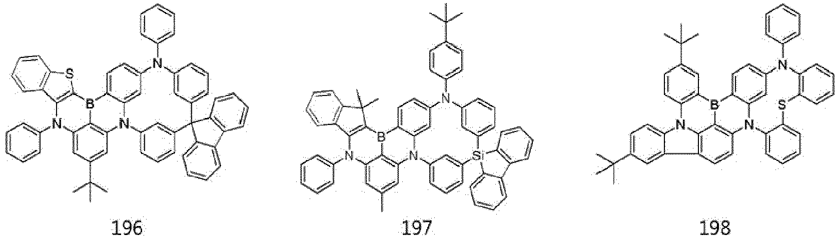

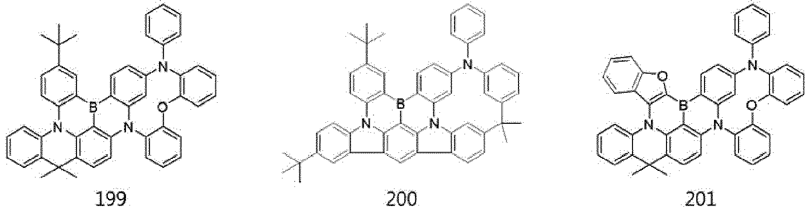

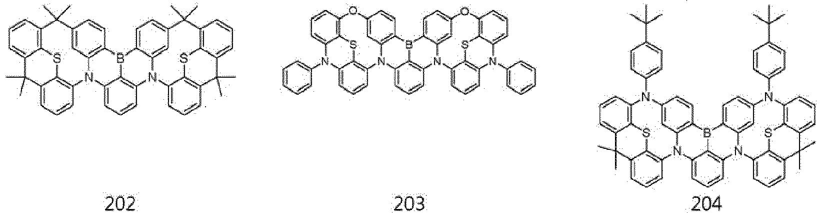

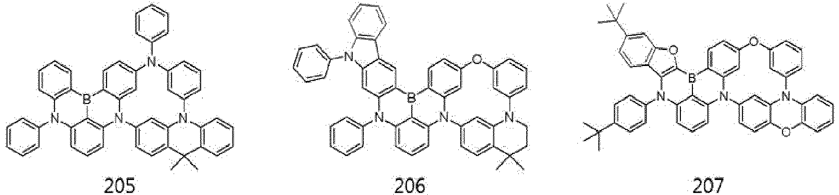

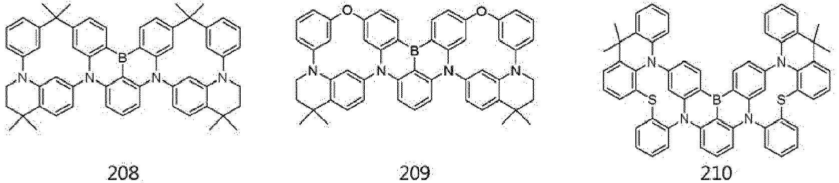

4. An organic electroluminescent device comprising a first electrode, a second electrode, and one or more organic layers interposed between the first and second electrodes wherein one of the organic layers comprises the organic compound represented by Formula I or II according to claim 1.

5. The organic electroluminescent device according to claim 4, wherein the organic layers comprise an electron injecting layer, an electron transport layer, a hole injecting layer, a hole transport layer, an electron blocking layer, a hole blocking layer, and/or a light emitting layer, at least one of which comprises the organic compound represented by Formula I or II.

6. The organic electroluminescent device according to claim 5, wherein the light emitting layer is composed of a host and the compound represented by Formula I or II as a dopant.

7. The organic electroluminescent device according to claim 6, wherein the host is an anthracene derivative represented by Formula C: 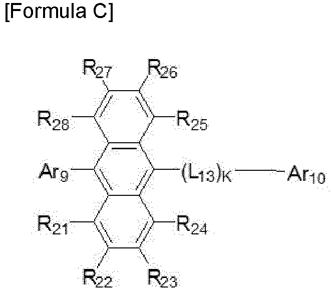 wherein R₂₁ to R₂₈ are identical to or different from each other and are as defined for R in Formula I or II, Ar₉ and Ar₁₀ are identical to or different from each other and are each independently selected from hydrogen, deuterium, substituted or unsubstituted C₁-C₃₀ alkyl, substituted or unsubstituted C₆-C₅₀ aryl, substituted or unsubstituted C₂-C₃₀ alkenyl, substituted or unsubstituted C₂-C₂₀ alkynyl, substituted or unsubstituted C₃-C₃₀ cycloalkyl, substituted or unsubstituted C₅-C₃₀ cycloalkenyl, substituted or unsubstituted C₂-C₅₀ heteroaryl, substituted or unsubstituted C₂-C₃₀ heterocycloalkyl, substituted or unsubstituted C₁-C₃₀ alkoxy, substituted or unsubstituted C₆-C₃₀ aryloxy, substituted or unsubstituted C₁-C₃₀ alkylthioxy, substituted or unsubstituted C₆-C₃₀ arylthioxy, halogen, substituted or unsubstituted amine, and substituted or unsubstituted silyl, L₁₃ is a single bond or is selected from substituted or unsubstituted C₆-C₂₀ arylene and substituted or unsubstituted C₂-C₂₀ heteroarylene, and k is an integer from 1 to 3, provided that when k is 2 or more, the linkers L₁₃ are identical to or different from each other.

8. The organic electroluminescent device according to claim 7, wherein Ar₉ in Formula C is represented by Formula C-1: 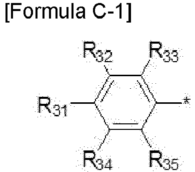 wherein R₃₁ to R₃₅ are identical to or different from each other and are as defined for R in Formula I or II and each of R₃₁ to R₃₅ is optionally bonded to an adjacent substituent to form a saturated or unsaturated ring.

9. The organic electroluminescent device according to claim 7, wherein the anthracene derivative represented by Formula C is selected from the compounds of Formulae C1 to C66:

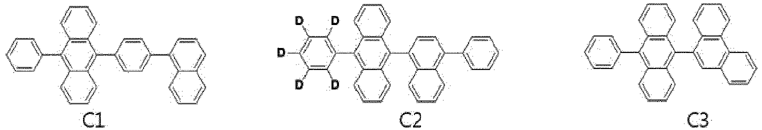

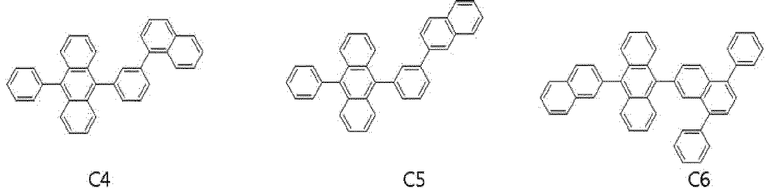

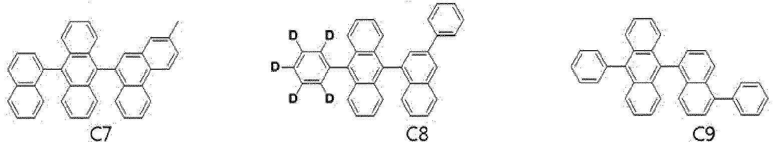

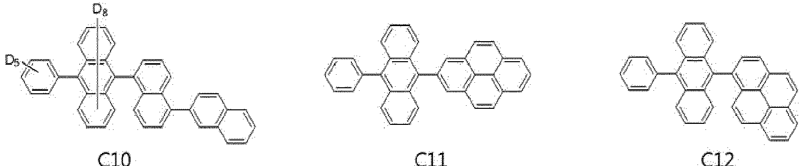

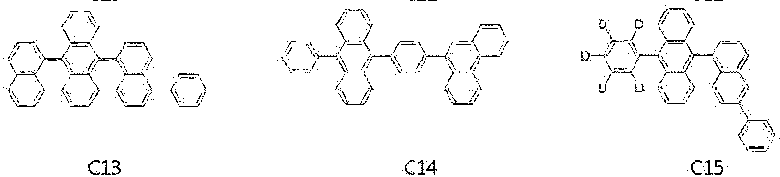

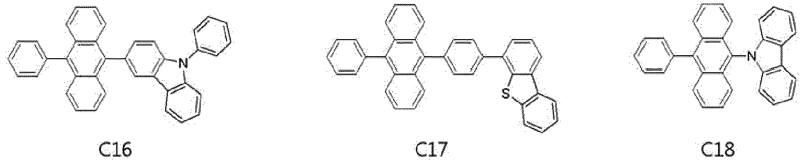

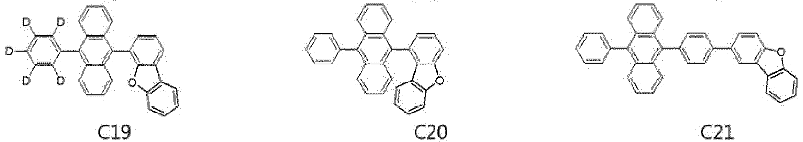

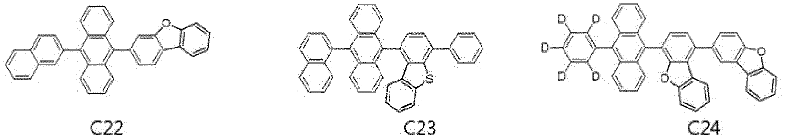

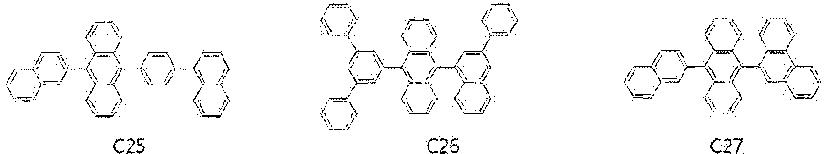

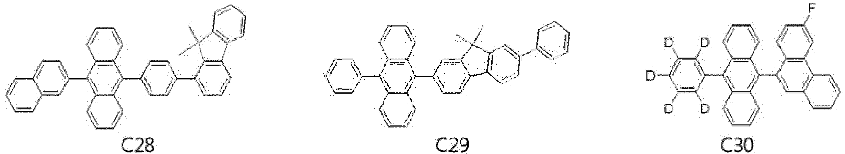

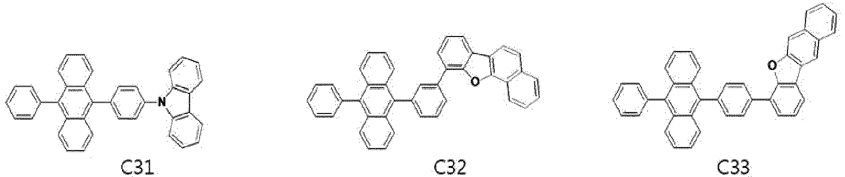

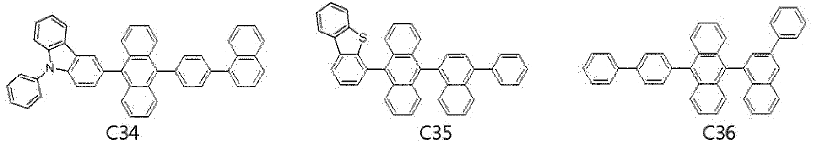

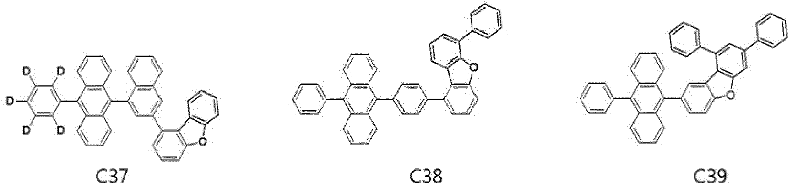

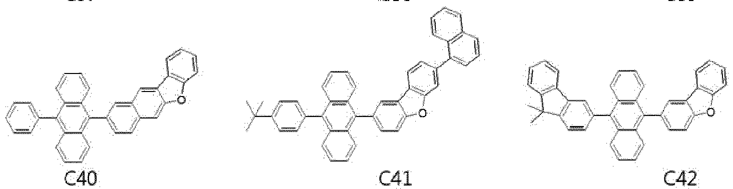

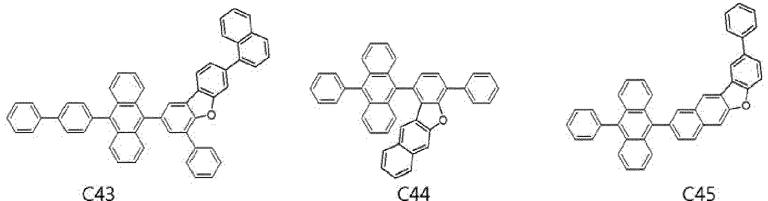

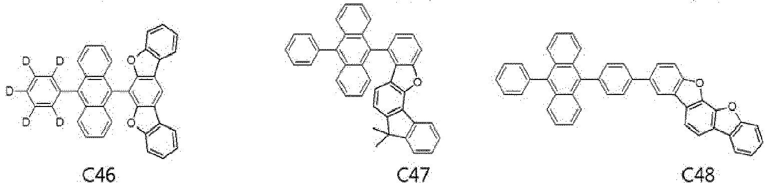

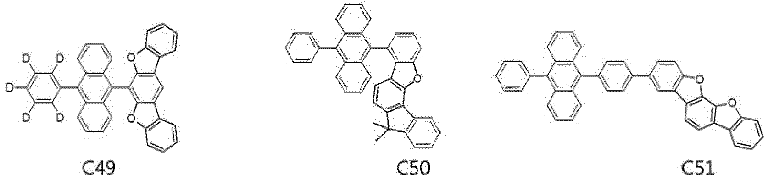

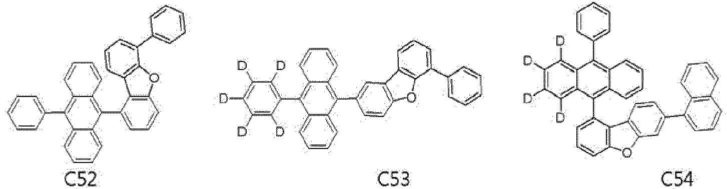

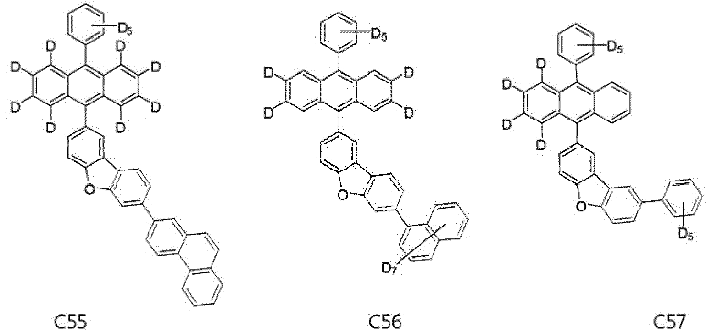

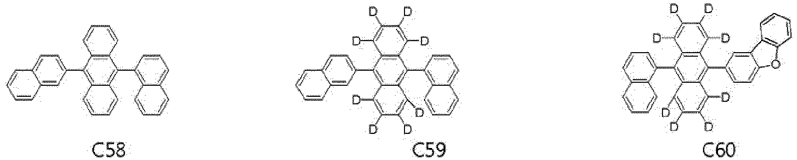

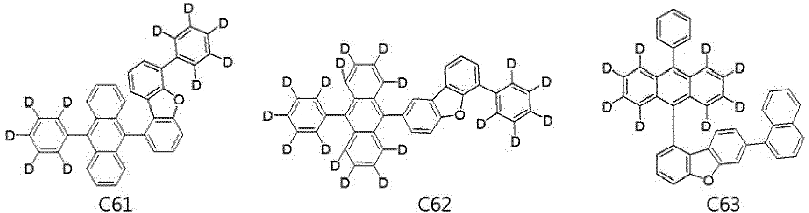

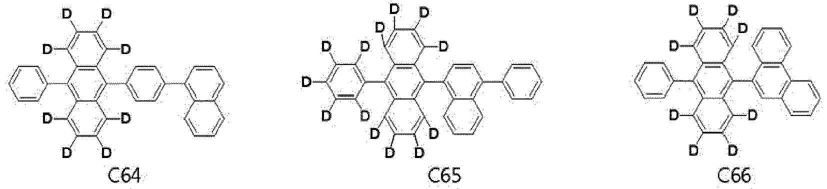

10. The organic electroluminescent device according to claim 5, wherein one or more of the layers are formed by a deposition or solution process.

11. The organic electroluminescent device according to claim 4, wherein the organic electroluminescent device is used in a display or lighting system selected from flat panel displays, flexible displays, monochromatic flat panel lighting systems, white flat panel lighting systems, flexible monochromatic lighting systems, and flexible white lighting systems.
